(19) Europäisches Patentamt
European Patent Office
Office européen des brevets

(11) Veröffentlichungsnummer: **0 046 451**
**A2**

(12) **EUROPÄISCHE PATENTANMELDUNG**

(21) Anmeldenummer: **81810329.3**

(22) Anmeldetag: **13.08.81**

(51) Int. Cl.³: **C 07 D 233/64**, C 07 D 263/32, C 07 D 401/04, C 07 D 401/14, C 07 D 403/04, C 07 D 403/14, C 07 D 405/04, C 07 D 405/14, C 07 D 409/04, C 07 D 409/14, C 07 D 413/04

(30) Priorität: **19.08.80 CH 6255/80**

(43) Veröffentlichungstag der Anmeldung: **24.02.82 Patentblatt 82/8**

(84) Benannte Vertragsstaaten: **AT BE CH DE FR GB IT LI LU NL SE**

(71) Anmelder: **CIBA-GEIGY AG, Patentabteilung Postfach, CH-4002 Basel (CH)**

(72) Erfinder: **Sallmann, Alfred, Dr., Joachimsackerstrasse 12, CH-4103 Bottmingen (CH)**

(54) **Neue Diaryl-Imidazol-Verbindungen, Verfahren zu ihrer Herstellung, diese enthaltende pharmazeutische Präparate und ihre Verwendung.**

(57) Die Erfindung betrifft neue Diaryl-Imidazol-Derivate, insbesondere Verbindungen der Formel I

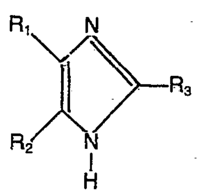

(I),

worin $R_1$ und $R_2$ unabhängig voneinander carbocyclisches Aryl und/oder Heteroaryl bedeuten, $R_3$ einen Rest der Formel $-alk-A-R_4$ darstellt, in der alk einen zweiwertigen Kohlenwasserstoffrest bedeutet, A eine Gruppe der Formel $-O-$ oder $-S(O)_n-$ darstellt und n 0, 1 oder 2 ist, und $R_4$ einen gegebenenfalls substituierten Kohlenwasserstoffrest oder, sofern n 0 ist, Wasserstoff bedeutet, und ihre Salze.

Die Verbindungen der Formel I, welche als externe Hautphlogostatika oder Antithrombotika verwendet werden können, werden nach an sich bekannten Verfahren hergestellt.

EP 0 046 451 A2

0046451

CIBA-GEIGY AG
Basel (Schweiz)

4-13019/+

# Neue Diaryl-Imidazol-Verbindungen, Verfahren zu ihrer Herstellung, diese enthaltende pharmazeutische Präparate und ihre Verwendung

Die Erfindung betrifft neue Diaryl-Imidazol-Derivate, insbesondere Verbindungen der Formel I

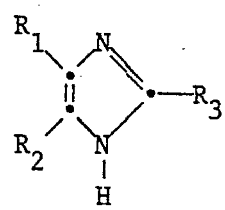

(I),

worin $R_1$ und $R_2$ unabhängig voneinander carbocyclisches Aryl und/oder Heteroaryl bedeuten, $R_3$ einen Rest der Formel -alk-A-$R_4$ darstellt, in der alk einen zweiwertigen Kohlenwasserstoffrest bedeutet, A eine Gruppe der Formel -O- oder $-S(O)_n-$ darstellt und n 0, 1 oder 2 ist, und $R_4$ einen gegebenenfalls substituierten Kohlenwasserstoffrest oder, sofern n 0 ist, Wasserstoff bedeutet, und ihre Salze, Verfahren zu ihrer Herstellung, ihre Verwendung, z.B. als Arzneimittelwirkstoffe, und pharmazeutische Präparate, die solche Verbindungen enthalten, sowie Formulierungsverfahren.

Carbocyclisches Aryl ist beispielsweise monocyclisches carbocyclisches Aryl, wie gegebenenfalls substituiertes Phenyl.

Heteroaryl ist beispielsweise monocyclisches, vorzugsweise 5- oder 6-gliedriges Heteroaryl, wobei mindestens ein Ringatom ein Heteroatom, wie ein Stickstoff-, Sauerstoff- oder Schwefelatom darstellt, und ein Ringstickstoff gegebenenfalls in oxidierter Form vorliegen kann. Solche 5-gliedrigen Reste sind z.B. Pyrrolyl, wie 2-Pyrrolyl, Furyl, wie 2-Furyl, Thienyl, wie 2- oder 3-Thienyl. Als 6-gliedriges Heteroaryl kommt z.B. Pyridyl, wie 2-, 3- oder 4-Pyridyl,

das auch N-oxidiert sein kann, wie 1-Oxido-pyridyl, z.B. 1-Oxido-3-pyridyl, und ebenfalls Pyrimidyl, wie 2-Pyrimidyl, in Frage, die gegebenenfalls substituiert sein können.

Als Substituenten von carboxyclischem Aryl bzw. Heteroaryl kommen beispielsweise Hydroxy, Halogen, Niederalkyl, Niederalkoxy und/oder Acyloxy in Frage, wobei Acyloxy beispielsweise von einer organischen Carbonsäure abgeleitet ist und z.B. Niederalkanoyloxy bedeutet.

Ein zweiwertiger Kohlenwasserstoffrest alk ist beispielsweise ein Niederalkylen- oder Niederalkylidenrest.

Ein gegebenenfalls substituierter Kohlenwasserstoffrest $R_4$ ist beispielsweise carbocyclisches Aryl, wie Phenyl, oder ein aliphatischer Kohlenwasserstoffrest, wie Niederalkyl, welcher gegebenenfalls durch Phenyl, Hydroxy, Niederalkoxy, Phenylniederalkoxy oder Niederalkanoyloxy substituiert sein kann, wobei Phenyl jeweils Hydroxy, Halogen, Niederalkyl, Niederalkoxy und/oder Niederalkanoyloxy als Substituenten aufweisen kann. Solche Reste $R_4$ sind z.B.: Phenyl, Niederalkyl, Phenylniederalkyl, Hydroxyniederalkyl, Niederalkoxyniederalkyl, Phenylniederalkoxyniederalkyl oder Niederalkanoyloxyniederalkyl.

Vor- und nachstehend sind unter "niederen" organischen Reste und Verbindungen vorzugsweise solche zu verstehen, die bis und mit 7, vor allem bis und mit 4 Kohlenstoffatome enthalten.

Die vor- und nachstehend verwendeten Allgemeindefinitionen haben in erster Linie die folgenden Bedeutungen:

Halogen ist z.B. Halogen bis und mit Atomnummer 35, wie Fluor, Chlor oder Brom, ferner Iod.

Niederalkyl ist z.B. Methyl, Ethyl, n-Propyl, Isopropyl, n-Butyl, Isobutyl, sec.-Butyl, tert.-Butyl, ferner ein Hexyl- oder

Heptylrest.

Niederalkoxy ist z.B. Methoxy, Ethoxy, n-Propyloxy, Isopropyloxy, n-Butyloxy, Isobutyloxy, sec-Butyloxy oder tert.-Butyloxy.

Niederalkanoyloxy ist z.B. Acetyl-, Propionyl-, Butyryl-, Isobutyryl- oder tert.-Butyryloxy.

Niederalkylen ist z.B. geradkettig, wie Ethylen, 1,3-Propylen oder 1,4-Butylen, oder verzweigt, wie 1,2-Propylen, 1,2- oder 1,3-(2-Methyl)-propylen oder 1,2-Butylen.

Niederalkyliden ist z.B. Methylen, Ethyliden oder 1,1- oder 2,2-Propyliden, ferner ein Butyliden.

Phenylniederalkyl ist z.B. Benzyl, 2-Phenylethyl, 2- oder 3-Phenylpropyl oder Triphenylmethyl.

Hydroxyniederalkyl ist z.B. Hydroxyethyl, Hydroxypropyl, 1,2-Dihydroxyethyl oder -propyl.

Niederalkoxyniederalkyl ist z.B.: Methoxyethyl, 2-Ethoxyethyl, 3-Methoxypropyl, 3-Ethoxypropyl oder 1,2-Dimethoxyethyl.

Phenylniederalkoxyniederalkyl ist z.B. 2-Benzyloxyethyl oder 2-(2-Phenylethoxy)-ethyl.

Niederalkanoyloxyniederalkyl ist z.B. Acetyloxymethyl, 2,2-Dimethyl-propionyloxymethyl, 2-Acetyloxyethyl oder 2,2-Dimethylpropionyloxyethyl (Pivaloyloxyethyl).

Salze von erfindungsgemässen Verbindungen der Formel I sind vorzugsweise pharmazeutisch verwendbare Salze, wie entsprechende Säureadditionssalze, oder, wenn mindestens einer der Reste $R_1$ und $R_2$ durch Hydroxy substituiert ist, Salze mit Basen. Geeignete Säureadditionssalze sind beispielsweise Salze mit anorganischen Säuren, wie

Mineralsäuren, mit Sulfaminsäuren, wie Cyclohexylsulfaminsäure, mit organischen Carbonsäuren, wie Niederalkancarbonsäuren, gegebenenfalls ungesättigten Dicarbonsäuren oder durch Hydroxy und/oder Oxo substituierte Carbonsäuren, oder mit Sulfonsäuren. Als Beispiele für solche Salze sind zu nennen: Sulfate oder Hydrohalogenide, wie Hydrobromide oder Hydrochloride, Acetate, Oxalate, Malate, Fumarate oder Maleinate, Tartrate, Pyruvate oder Citrate, Sulfonate, wie Methan-, Benzol- oder p.Toluolsulfonate.

Geeignete Salze mit Basen sind beispielsweise Alkali- oder Erdalkalimetallsalze, wie Natrium-, Kalium- oder Magnesiumsalze, pharmazeutisch verwendbare Uebergangsmetallsalze, wie Zink- oder Kupfersalze, oder Salze mit Ammoniak oder von substituierten organischen Aminen, wie Morpholin, Thiomorpholin, Piperidin, Pyrrolidin, von Mono-, Di- bzw. Triniederalkylaminen, wie Ethyl- oder tert.-Butylamin, Diethyl- oder Diisopropylamin oder Triethylamin, oder von Mono-, Di- bzw. Trihydroxyniederalkylaminen, wie Mono-, Di- bzw. Triethanolamin.

Die neuen Verbindungen der Formel I und ihre pharmazeutisch verwendbaren Salze weisen wertvolle pharmakologische Eigenschaften auf. Insbesondere besitzen sie, z.B. bei lokaler Anwendung, eine ausgeprägte antiinflammatorische Wirkung.

Diese Eigenschaft lässt sich beispielsweise nach der von G. Tonelli, L. Thibault, Endocrinology 77, 625 (1965) entwickelten Methode durch Hemmung des mit Crotonöl induzierten Rattenohroedems bei der Normalratte im Dosisbereich von etwa 1 bis etwa 100 mg/ml nachweisen.

Die ausgezeichnete entzündungshemmende Wirkung lässt sich ebenfalls im Ultraviolettstrahlen-Dermatitis-Hemmtest am Meerschweinchen [Methodik: Weirich, E.G.; Longauer, J.; Kirkwood, A.H.; Dermatologica 152, 87-99 (1976)] und mit Hilfe des Crotonöl-Dermatitis-Hemmtests am Kaninchen [Methodik: Weirich, E.G.; Longauer, J.; Kirkwood, A.H.; Arch. Derm. Res. 259, 141-9 (1977)] jeweils im Dosisbereich von etwa 0,01 bis etwa 1,0 % G/G bei topischer Applikation einer entsprechenden Lösung erfassen. Weiterhin zeigen die erfindungsgemässen Verbindungen im Hyperplasie-Hemmtest am Meerschweinchen im Dosisbereich von etwa 0,01 bis 1,0 % G/G bei topischer Anwendung eine ausgeprägte Hemmwirkung [Methodik: Weirich, E.G.; Longauer, J.; Kirkwood, A.H.; Dermatologica 151, 321-332 (1975)]. Ausserdem wurden die Substanzen einer Aktivitätsprüfung am Menschen unterzogen. Im Hautvasokonstriktions-Lösungs-Test konnte im Dosisbereich von etwa $1 \cdot 10^{-5}$ bis $1 \cdot 10^{-1}$ G/G ein beträchtlicher Vasokonstriktionseffekt festgestellt werden [Methodik: Weirich, E.G.; Lutz, U.; Dermatologica 155, 328-334 (1977)].

Weiterhin zeichnen sich die erfindungsgemässen Verbindungen durch deutliche Effekte gegen einige Virusstämme aus. So wird beispielsweise bei Experimenten am Meerschweinchen, die mit HVH 2/Angelotti infiziert sind [Methodik: B. Lukas et al., Arch. Ges. Virusforsch. 44, 153-5 (1974) und 49, 1-11 (1975)], nach 2 mal täglicher intravaginaler Applikation über 5 Tage von 0,1 ml eines Gels mit 0,2%iger Konzentration eine rasche Regression bzw. völlige Reduktion der durch Herpes genitalis verursachten Symptome festgestellt.

Die erfindungsgemässen Verbindungen der Formel I sind daher als Arzneimittel, insbesondere externe (topische) Hautphlogistatika für die Behandlung entzündlicher Dermatosen jeglicher Genese, wie bei leichten Hautirritationen, Kontaktdermatitis, Exanthemen, Verbrennungen, sowie als Schleimhautphlogostatika für die Behandlung von Mukosaentzündungen, z.B. der Augen, Nase, Lippen, Mund, Genital-, Analregion, geeignet. Ferner können die Verbindungen als Sonnenschutzmittel sowie als antivirale Mittel, z.B. Anti-Herpes-Mittel, verwendet werden.

Die vorliegende Erfindung betrifft ebenfalls die Verwendung der erfindungsgemässen Verbindungen und deren Salze sowie pharmazeutische Präparate, die diese enthalten, und deren Verwendung zur Behandlung von Entzündungen, z.B. von entzündlichen Erkrankungen unterschiedlichster Genese, sowie zur Herstellung von Arzneimitteln.

Verbindungen der Formel I, worin A eine Gruppe der Formel $-S(O)_n-$ und n 0, 1 oder 2 ist, hemmen die durch eine Arthus-Reaktion nach K.O. Buttler et al [Thrombosis Research 15, 314-340 (1979)] ausgelöste Aktivität der Blutplättchen in Dosen von etwa 30 bis etwa 300 mg/kg p.o. und können daher ebenfalls als Antithrombotika verwendet werden.

Die Erfindung betrifft beispielsweise Verbindungen der Formel I, worin $R_1$ und $R_2$ unabhängig voneinander Phenyl-, Pyrrolyl-, Furyl-, Thienyl-, Pyridyl-, 1-Oxido-pyridyl- oder Pyrimidylreste bedeuten, die jeweils unsubstituiert oder durch Hydroxy, Halogen, Niederalkyl, Niederalkoxy und/oder Niederalkanoyloxy substituiert sind, $R_3$ eine Gruppe der Formel $-alk-A-R_4$ darstellt, in der alk Niederalkylen oder Niederalkyliden bedeutet, A eine Gruppe der Formel -O- oder $-S(O)_n-$ darstellt und n 0, 1 oder 2 ist und $R_4$ einen Phenylrest, Niederalkyl, durch einen Phenylrest, Hydroxy, Niederalkoxy, einen Phenylniederalkoxyrest und/oder Niederalkanoyloxy substituiertes Niederalkyl oder, sofern n 0 ist, Wasserstoff bedeutet, wobei ein Phenylrest jeweils unsubstituiert oder durch Hydroxy, Halogen, Niederalkyl, Niederalkoxy und/oder Niederalkanoyloxy substituiert ist, sowie ihre Salze, insbesondere pharmazeutisch verwendbare Salze.

Die Erfindung betrifft beispielsweise Verbindungen der Formel I, worin $R_1$ und $R_2$ unabhängig voneinander Phenyl-, Pyridyl-, wie 3-Pyridyl- oder 1-Oxido-pyridylreste, wie 1-Oxido-3-pyridyl, bedeuten, die jeweils unsubstituiert oder durch Hydroxy, Halogen mit Atomnummer bis und mit 35, wie Chlor, Niederalkyl mit bis und mit 4 C-Atomen, wie Methyl, Niederalkoxy mit bis und mit 4 C-Atomen, wie Methoxy, und/oder Niederalkanoyloxy mit bis und mit 5 C-Atomen, wie Acetyloxy, substituiert sind, $R_2$ eine Gruppe der Formel $-alk-A-R_4$ darstellt, in der alk Niederalkylen mit bis und mit 4 C-Atomen, wie Ethylen, oder Niederalkyliden mit bis und mit 4 C-Atomen, wie Methylen, bedeutet, A eine Gruppe der Formel -O- oder $-S(O)_n-$ darstellt und n 0, 1 oder 2 ist und $R_4$ einen Phenylrest, Niederalkyl mit

bis und mit 4 C-Atomen, wie Methyl, durch einen Phenylrest, Hydroxy, Halogen mit Atomnummer bis und mit 35, wie Chlor, Niederalkoxy mit bis und mit 4 C-Atomen, wie Methoxy, einen Phenylniederalkoxyrest mit bis und mit 4 C-Atomen im Alkylteil, wie 2-Phenylethoxy, und/oder Niederalkanoyloxy mit bis und mit 5 C-Atomen, wie Acetyloxy, substituiertes Niederalkyl mit bis und mit 4 C-Atomen, wie Methyl, wobei ein Phenylrest jeweils unsubstituiert oder durch Hydroxy, Niederalkyl mit bis und mit 4 C-Atomen, wie Methyl, Niederalkoxy mit bis und mit 4 C-Atomen, wie Methoxy, Halogen mit Atomnummer bis und mit 35, wie Chlor, und/oder Niederalkanoyloxy mit bis und mit 5 C-Atomen, wie Acetyloxy, substituiert ist, oder, sofern n 0 ist, Wasserstoff darstellt, sowie ihre Salze, insbesondere pharmazeutisch verwendbare Salze.

Die Erfindung betrifft beispielsweise Verbindungen der Formel I, worin einerseits $R_1$ und $R_2$ durch Niederalkoxy mit bis und mit 4 C-Atomen, wie Methoxy, substituiertes Phenyl bedeuten oder worin andererseits einer der Reste $R_1$ und $R_2$ Phenyl oder durch Hydroxy, Niederalkoxy mit bis und mit 4 C-Atomen, wie Methoxy, oder Halogen mit Atomnummer bis und mit 35, wie Chlor, substituiertes Phenyl und der andere Pyridyl, wie 3-Pyridyl, durch Hydroxy oder Niederalkoxy mit bis und mit 4 C-Atomen, wie Ethoxy, substituiertes Pyridyl, wie 3-Pyridyl, oder 1-Oxido-pyridyl, wie 1-Oxido-3-pyridyl, darstellt, $R_3$ einen Rest der Formel $-alk-A-R_4$ bedeutet, in der alk Niederalkylen mit bis und mit 4 C-Atomen, wie Ethylen, oder geradkettiges bzw. verzweigtes Niederalkyliden mit bis und mit 4 C-Atomen, wie Methylen oder 1,1-Ethyliden, darstellt, A eine Gruppe der Formel -O- oder $-S(O)_n-$ bedeutet und n 0, 1 oder 2 ist, und $R_4$ Phenyl, Niederalkyl mit bis und mit 4 C-Atomen, wie Methyl, oder, sofern A ein Sauerstoffatom bedeutet, Wasserstoff darstellt, sowie ihre Salze, insbesondere pharmazeutisch verwendbare Salze.

Die Erfindung betrifft insbesondere Verbindungen der Formel I, worin $R_1$ und $R_2$ jeweils durch Niederalkoxy mit bis und mit 4 C-Atomen, wie

Methoxy, substituiertes Phenyl bedeuten, $R_3$ einen Rest der Formel $-alk-A-R_4$ darstellt, in der alk Methylen oder geradkettiges Niederalkylen mit bis und mit 4 C-Atomen, wie Ethylen, bedeutet, A eine Gruppe der Formel $-S(O)_n$ darstellt, und n 0, 1 oder 2 ist, und $R_4$ Phenyl darstellt, sowie ihre Salze, insbesondere pharmazeutisch verwendbare Salze.

Die Erfindung betrifft in erster Linie Verbindungen der Formel I, worin einerseits $R_1$ und $R_2$ durch Niederalkoxy mit bis und mit 4 C-Atomen, wie Methoxy, substituiertes Phenyl bedeuten oder worin andererseits einer der Reste $R_1$ und $R_2$ Phenyl oder durch Halogen mit Atomnummer bis und mit 35, wie Chlor, substituiertes Phenyl und der andere Pyridyl, wie 3-Pyridyl, darstellt, $R_3$ einen Rest der Formel $-alk-A-R_4$ darstellt, in der alk Methylen oder Ethylen bedeutet, A eine Gruppe der Formel -O- oder $-S(O)_n$ und n 0 oder 1 ist, und $R_4$ Phenyl oder Niederalkyl mit bis und mit 4 C-Atomen, wie Methyl, darstellt, sowie ihre Salze, insbesondere pharmazeutisch verwendbare Salze.

Die Erfindung betrifft namentlich die in den Beispielen genannten Verbindungen und ihre Salze, insbesondere pharmazeutisch verwendbare Salze von solchen Verbindungen mit salzbildenden Gruppen, sowie die in den Beispielen aufgeführten Herstellungsverfahren.

Die Erfindung betrifft namentlich die in den Beispielen genannten Verbindungen der Formel I und ihre Salze, insbesondere pharmazeutisch verwendbare Salze von solchen Verbindungen mit salzbildenden Gruppen.

Die neuen Verbindungen der Formel I oder deren Salze lassen sich in an sich bekannter Weise herstellen.

Eine Verfahrensweise besteht beispielsweise darin, dass man aus einer Verbindung der Formel

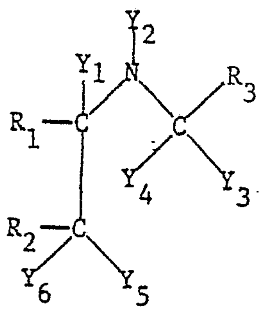

(II) ,

worin einer der Reste $Y_1$ und $Y_6$ Hydroxy oder Amino und der andere sowie $Y_2$ Wasserstoff darstellt und $Y_3$ gemeinsam mit $Y_4$ und $Y_5$ eine Gruppe der Formel =N- bedeutet oder worin $Y_1$ gemeinsam mit $Y_6$ eine Bindung darstellt, $Y_2$ Wasserstoff ist, $Y_3$ Hydroxy oder Amino bedeutet und $Y_4$ gemeinsam mit $Y_5$ eine Gruppe der Formel -NH- darstellt oder worin $Y_1$ gemeinsam mit $Y_6$ eine Bindung darstellt, $Y_2$ gemeinsam mit $Y_3$ eine zusätzliche Bindung und einer der Reste $Y_4$ und $Y_5$ Amino und der andere Amino, Hydroxy oder reaktionsfähiges verestertes Hydroxy, insbesondere Halogen oder Sulfonyloxy, darstellt oder worin $Y_1$ Hydroxy ist, $Y_2$ sowie $Y_3$ Wasserstoff bedeutet, $Y_4$ Hydroxy oder Amino darstellt und $Y_5$ gemeinsam mit $Y_6$ eine Gruppe der Formel =NH oder, sofern $Y_4$ Amino ist, Oxo oder Imino bedeutet, oder einem Tautomeren und/oder Salz davon, unter Einführung einer gegebenenfalls zusätzlichen Bindung H-Z abspaltet, und gewünschtenfalls die verfahrensgemäss erhältliche freie Verbindung in ein Salz oder ein verfahrensgemäss erhältliches Salz in die freie Verbindung oder in ein anderes Salz überführt und/oder, wenn erwünscht, ein erfindungsgemäss erhältliches Gemisch von isomeren Verbindungen der Formel I in die einzelnen Isomeren auftrennt.

Dabei bedeutet Z Hydroxy oder Amino $Y_1$ bzw. $Y_6$, $Y_3$ oder $Y_4$ bzw. $Y_5$ oder Halogen bzw. Sulfonyloxy $Y_4$ bzw. $Y_5$.

Reaktionsfähiges verestertes Hydroxy ist beispielsweise mit einer anorganischen Mineralsäure, wie Halogenwasserstoffsäure, oder mit einer organischen Sulfonsäure, wie Niederalkan- oder gegebenenfalls substituierte Benzolsulfonsäure, verestertes Hydroxy und bedeutet in erster Linie Halogen, z.B. Chlor oder Brom, sowie Sulfonyloxy, z.B. Methan- oder p-Toluolsulfonyloxy.

Tautomere von Verbindungen der Formel II sind beispielsweise solche, in denen eine partielle Enol- bzw. Enamin-Gruppierung der Formel

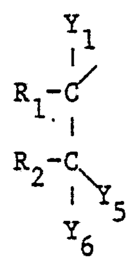

(IIa) ,

worin $Y_1$ gemeinsam mit $Y_6$ eine zusätzliche Bindung darstellt und $Y_5$ Hydroxy bzw. Amino bedeutet, in Form der entsprechenden tautomeren Keto- bzw. Ketiminform, worin $Y_1$ Wasserstoff ist und $Y_5$ gemeinsam mit $Y_6$ Oxo bzw. Imino bedeutet, vorliegt und/oder solche, in denen eine partielle Enol- bzw. Enamingruppierung der Formel

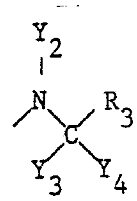

(IIb) ,

worin $Y_2$ gemeinsam mit $Y_3$ eine Bindung darstellt und $Y_4$ Hydroxy bzw. Amino bedeutet, in Form der entsprechenden tautomeren Form, worin $Y_2$ Wasserstoff ist und $Y_3$ gemeinsam mit $Y_4$ Oxo bzw. Imino darstellt, vorliegt, wobei die genannten Tautomeren miteinander im Gleichgewicht stehen.

Die Abspaltung von H - Z aus einer Verbindung der Formel II, einem Tautomeren und/oder Salz davon erfolgt in üblicher, insbesondere in der aus der Literatur für analoge Reaktionen bekannten Weise, erforderlichenfalls unter Erwärmen, wie in einem Temperaturbereich von etwa 20° bis etwa 250°C, unter Druck und/oder in Gegenwart eines katalytischen Mittels, vorzugsweise einer Säure. Als Säuren eignen sich beispielsweise anorganische Säuren, wie Mineralsäuren, z.B. Schwefelsäure, Polyphosphorsäure oder Halogenwasserstoffsäure, wie Chlorwasserstoffsäure, oder organische Säuren, wie Niederalkancarbonsäuren, z.B. Essigsäure. Dabei arbeitet man erforderlichenfalls in einem inerten Lösungsmittel, beispielswisse einem gegebenenfalls halogenierten Kohlenwasserstoff, wie Chloroform, Chlorbenzol, Hexan oder Toluol, einem Niederalkanol, wie Methanol oder Ethanol, einem Carbonsäureamid, wie Niederalkancarbonsäureamid, z.B. Dimethylformamid oder Formamid, oder einer Niederalkancarbonsäure, wie Ameisen- oder Essigsäure, und/oder unter Inertgas, wie Stickstoff.

Die Ausgangsstoffe der Formel II, ihre Tautomeren und/oder Salze werden nach an sich bekannten Verfahren zum überwiegenden Teil <u>in situ</u> gebildet und unter den Reaktionsbedingungen ohne Isolierung weiter zu der Verbindung der Formel I umgesetzt. Dabei kann die Abspaltung von H - Z unter direkter Cyclisierung oder im Anschluss an eine vorgelagerte Cyclisierung erfolgen.

So kann man in einer bevorzugten Ausführungsform des vorstehenden Verfahrens beispielsweise das Diketon der Formel

$$\begin{array}{l} R_1 - C = O \\ \quad\;\;\; | \\ R_2 - C = O \end{array} \qquad \text{(IIIa)}$$

mit einem Aldehyd der Formel $R_3$-C(=O)-H (IIIb) oder einem Salz davon, und einem Ueberschuss an Ammoniak unter Erwärmen umsetzen. Dabei wird beispielsweise intermediär eine Verbindung der Formel II, z.B. eine solche, worin $Y_1$ Hydroxy, $Y_2$ sowie $Y_6$ Wasserstoff bedeutet und $Y_3$ gemeinsam mit $Y_4$ und $Y_5$ eine Gruppe der Formel =N- darstellt, z.B.

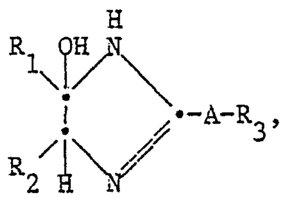 oder eine tautomere Form davon gebildet,

die unter den Reaktionsbedingungen erfindungsgemäss weiterreagiert.

Ferner kann man in weiteren bevorzugten Ausführungsformen des vorstehend beschriebenen Verfahrens ein acyliertes $\alpha$-Aminoketon der Formel

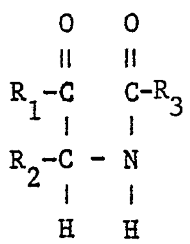

(IIIc)

oder ein Salz davon mit Ammoniak umsetzen. Dabei arbeitet man beispielsweise unter Erwärmen, z.B. in einem Temperaturbereich von etwa 50° bis etwa 250°C, und unter inerten Bedingungen.

Die Ausgangsstoffe der Formel (IIIc) sind bekannt oder werden nach an sich bekannten Verfahren hergestellt. Beispielsweise geht man von einer Verbindung der Formel

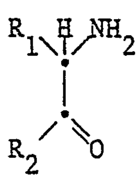

(IIId)

oder einem Salz davon aus und setzt diese mit einem Säurederivat der Formel $R_3$-COOH (IIIe), beispielsweise einem entsprechenden Anhydrid, wie einer Halogencarbonylverbindung, um.

In einer weiteren, besonders bevorzugten Variante des eingangs beschriebenen Verfahrens setzt man eine Verbindung der Formel

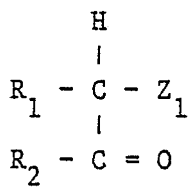

(IIIf)

worin $Z_1$ reaktionsfähiges verestertes Hydroxy bedeutet, oder ein Salz davon mit einer Verbindung der Formel

$$R_3 - Z_2 \qquad \text{(IIIg)},$$

worin $Z_2$ ein Amidinorest oder Ammoniumcarboxylat bedeutet, oder ein Salz davon gegebenenfalls mit Ammoniak um.

Die Umsetzung mit einem Amidin der Formel (IIIg) erfolgt üblicherweise unter Erwärmen, beispielsweise in einem Temperaturbereich von etwa 50° bis etwa 250°C.

Die Reaktion des Ammoniumcarboxylats der Formel (IIIg) mit einer Verbindung der Formel (IIIf) wird mit einem mindestens 3-molaren, oder, falls die Verbindung der Formel (IIIf) in Salzform vorliegt, mindestens 4-molaren Ueberschuss des Ammoniumsalzes der Verbindung der Formel (IIIg), gegebenenfalls unter Erwärmen, z.B. in einem Temperaturbereich von etwa 50° bis etwa 250°C, vorzugsweise bei 90° bis 120°C, durchgeführt, wobei die Verbindung der Formel (IIIg) gleichzeitig als Lösungsmittel dienen kann. Diese Variante kann beispielsweise auch dahingehend abgewandelt werden, dass man das Ammoniumsalz der Formel (IIIg), in bezug auf den reaktionsfähigen Ester $Z_1$, in etwa äquimolarer Menge einsetzt und zusätzlich Ammoniak, gegebenenfalls in Form eines Salzes einer gegenüber $R_3$-COOH schwächeren Säure, in überschüssiger Menge, vorzugsweise in 3- bis 5-fachem Ueberschuss, zugibt.

Eine reaktionsfähige veresterte Hydroxygruppe $Z_1$ ist beispielsweise eine vorzugsweise durch starke anorganische oder organische Säuren, wie starke Mineralsäuren, z.B. Halogenwasserstoffsäuren, wie Chlor- oder Bromwasserstoffsäure, oder starke organische Sulfonsäuren, wie entsprechende Niederalkan- oder Arylsulfonsäuren, z.B. Methan- oder

eine gegebenenfalls substituierte Benzolsulfonsäure, veresterte Hydroxygruppe und stellt z.B. Halogen, wie Chlor oder Brom, Niederalkylsulfonyloxy, z.B. Methyl- oder Ethylsulfonyloxy, oder Arylsulfonyloxy, z.B. p-Toluol- oder Benzolsulfonyloxy, dar.

Das Ammoniumsalz der Formel (IIIg) kann auch unter den Reaktionsbedingungen <u>in situ</u> gebildet werden, beispielsweise indem man im Reaktionsgemisch die freie Säure der Formel (IIIg) vorlegt und mit flüssigem oder gasförmigem Ammoniak versetzt. Bei dieser Ausführungsform kann das Ammoniak auch in Form eines Salzes mit einer gegenüber $R_3$-COOH schwächeren Säure, wie Kohlensäure, zugegeben werden.

Als geeignete Lösungsmittel kommen z.B. gegebenenfalls halogenierte Kohlenwasserstoffe, wie gegebenenfalls halogenierte aliphatische, cycloaliphatische oder aromatische Kohlenwasserstoffe, wie Hexan, Cyclohexan, Toluol, Chloroform, oder Chlorbenzol, Alkanole, wie Propanol, Isopropanol, Butanole, Pentanole oder Octanole, Ether, wie Dimethoxyethan, Ethylenglykolmonoethylether, Dioxan oder Tetrahydrofuran, Niederalkancarbonsäuren, wie Ameisen- oder Essigsäure oder vorzugsweise Säuren der Formel (IIIe), Amide, wie Niederalkancarbonsäureamide, z.B. Formamid oder Dimethylformamid, sowie Lactame, z.B. N-Methylpyrrolidon, Sulfoxide, wie Dimethylsulfoxid, oder Wasser in Frage.

Eine bevorzugte Ausführung dieser Variante zur erfindungsgemässen Darstellung von Verbindungen der Formel I über Verbindungen der Formel II besteht darin, dass man eine Verbindung der Formel (IIIf), worin $Z_1$ beispielsweise Halogen, wie Brom, bedeutet, mit einem Ammoniumsalz der Formel (IIIg) bei einer Reaktionstemperatur von etwa 100°C umsetzt. Die Verbindung der Formel (IIIg) wird im Ueberschuss zugegeben, beispielsweise in einem Verhältnis gegenüber dem Ester der Formel (IIIf) von etwa 4:1 bis etwa 6:1, und lässt sich <u>in situ</u> bilden, indem man z.B. die entsprechende Säure unter den Reaktionsbedingungen mit flüssigem Ammoniak umsetzt.

Die Ausgangsstoffe der Formel (IIIf) sind bekannt oder können nach an sich bekannten Verfahren hergestellt werden.

So lassen sie sich beispielsweise durch Esterkondensation von veresterten Säuren der Formeln $R_1-CH_2-COOH$ bzw. $R_2-CH_2-COOH$ mit veresterten Säuren der Formeln $R_1-COOH$ bzw. $R_2-COOH$, vorzugsweise in Gegenwart einer Base erhalten. Das resultierende α-Methylenketon der Formel

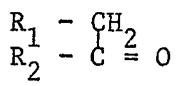

(IIIh)

wird beispielsweise bromiert und somit in eine Verbindung der Formel (IIIf) bzw. ein Salz, wie Hydrobromid, davon übergeführt, worin $Z_1$ Brom bedeutet.

Ausserdem kann man in einer weiteren bevorzugten Ausführungsform ein Oxazol der Formel

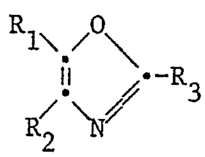

(IIIi)

mit Ammoniak über Verbindungen der Formel II zu Verbindungen der Formel I umsetzen.
Diese Reaktion wird gegebenenfalls unter Druck, bespielsweise bei 185 atü, und/oder Erwärmen, z.B. auf etwa 100° bis etwa 250°C, durchgeführt.

Die Verbindungen der Formel (IIIi) ihrerseits lassen sich nach an sich bekanntem Verfahren herstellen, beispielsweise durch Umsetzung von Verbindungen der Formel

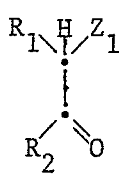

(IIIf) ,

worin $Z_1$ gegebenenfalls reaktionsfähiges verestertes Hydroxy bedeutet, mit einer Carbonsäure der Formel $R_3-COOH$, einem Salz davon oder einem funktionellen Derivat davon, wie einem entsprechenden Anhydrid, z.B. einem Halogencarbonylderivat, gegebenenfalls über eine erhältliche Zwischenverbindung der Formel

$R_2-C(=O)-C(R_1)-O-C(=O)-R_3$ (IIIk) und mit Ammoniak.

Diese Umsetzung erfolgt gegebenenfalls unter Druck und/oder Erwärmen, z.B. bei der Siedetemperatur des Lösungsmittels.

Bei den vorstehenden Varianten wird jeweils eine Verbindung der Formel II gebildet, die erfindungsgemäss, insbesondere in situ, zu einer Verbindung der Formel I weiterreagiert.

Einige der vorstehend beschriebenen Verfahrensvarianten lassen sich durch Anwendung milder Bedingungen so durchführen, dass die Verbindungen der Formel II bzw. ihre Tautomeren und/oder Salze isoliert werden können.

Verbindungen der Formel I oder Salze davon kann man weiterhin herstellen, indem man beispielsweise eine Verbindung der Formel

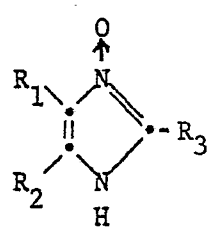

(IV)

oder ein Salz davon zu einer Verbindung der Formel I reduziert und gewünschtenfalls die verfahrensgemäss erhältliche freie Verbindung in ein Salz oder ein verfahrensgemäss erhältliches Salz in die freie Verbindung oder in ein anderes Salz überführt.

Die Reduktion erfolgt nach an sich bekannten Verfahren. So behandelt man Verbindungen der Formel (IV) oder deren Salze mit Wasserstoff in Gegenwart eines Hydrierungskatalysators oder mit einem Dithionit, z.B. Natriumdithionit, oder mit einem Phosphorhalogenid, z.B. Phosphortrichlorid. Als Hydrierungskatalysatoren lassen sich beispielsweise Elemente der VIII. Nebengruppe sowie Derivate davon, wie Platin, Palladium oder Palladiumchlorid, die gegebenenfalls auf einem üblichen Trägermaterial, wie Aktivkohle oder Erdalkalimetallverbindungen, z.B. Bariumcarbonat, aufgezogen sind, oder Raney-Nickel, verwenden.

Die Reduktion lässt sich erforderlichenfalls unter Kühlen oder Erwärmen, beispielsweise in einem Temperaturbereich von etwa 0° bis etwa 150°C, in einem inerten Lösungsmittel, wie einem halogenierten Kohlenwasserstoff, z.B. Chloroform, Tetrachlorkohlenstoff oder Chlorbenzol, oder einem Ether, wie Dimethoxyethan, Diethylether, Dioxan oder Tetrahydrofuran, und/oder unter Inertgas, z.B. Stickstoff, durchführen.

Die Ausgangsstoffe der Formel IV oder deren Salze sind in an sich bekannter Weise erhältlich, beispielsweise in dem eine Verbindung der Formel

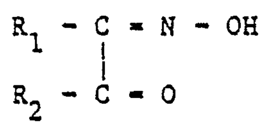 (IVa),

ein Tautomeres oder ein Salz davon <u>in situ</u> mit einem Ueberschuss an Ammoniak und einem Aldehyd der Formel $R_3$-C(=O)-H (IIIb) bei erhöhter Temperatur umsetzt.

Verbindungen der Formel I, worin $R_3$ einen Rest der Formel -alk-A-$R_4$ und A ein Sauerstoff- oder Schwefelatom bedeutet, lassen sich ferner herstellen, indem man in einer Verbindung der Formel

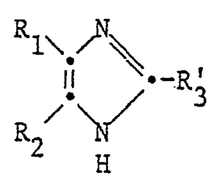 (V),

worin $R_3'$ einen in $R_3$ überführbaren Rest bedeutet, oder ein Salz davon den Rest $R_3'$ in einen Rest $R_3$ überführt und gewünschtenfalls die verfahrensgemäss erhältliche freie Verbindung in ein Salz oder ein verfahrensgemäss erhältliches Salz in die freie Verbindung oder in ein anderes Salz überführt.

In Reste $R_3$ überführbare Reste $R_3'$ sind beispielsweise Gruppen der Formel $-alk-R_4'$, worin $R_4'$ gegebenenfalls funktionell abgewandeltes Carboxy, wie verestertes oder anhydrisiertes Carboxy, gegebenenfalls acetalisiertes Formyl, oder funktionell abgewandeltes Hydroxy, wie verestertes oder verethertes Hydroxy, bedeutet.

Dabei ist beispielsweise unter verestertem Carboxy mit einem gegebenenfalls substituierten Niederalkanol, wie Methanol, oder 3- bis 8-gliedrigen Cycloalkanol, wie Cyclohexanol, verestertes Carboxy zu verstehen, wie gegebenenfalls substituiertes Niederalkoxycarbonyl, z.B. Ethoxycarbonyl, oder Cycloalkoxycarbonyl, z.B. Cyclohexyloxycarbonyl. Anhydrisiertes Carboxy ist beispielsweise mit einer Mineralsäure, wie Halogenwasserstoff-, z.B. Chlorwasserstoffsäure, anhydridisiertes Carboxy, wie Halogen-, z.B. Chlorcarbonyl. Unter acetalisiertem Formyl ist beispielsweise mit einem Niederalkanol, wie Methanol, oder Niederalkandiol, wie Glykol, acetalisiertes Formyl zu verstehen, wie Diniederalkoxy-, z.B. Dimethoxymethyl, oder Niederalkylendioxy-, z.B. Ethylendioxymethyl. Verestertes Hydroxy ist gegebenenfalls mit einer Niederalkancarbonsäure, wie Essigsäure, mit einem Kohlensäurehalbester, wie tert.-Butyl- oder Benzylkohlensäure, mit einer Mineralsäure, wie Schwefelesäure oder Halogenwasserstoff-, z.B. Chlorwasserstoffsäure, oder mit einer organischen Sulfonsäure, wie Methan- oder p-Toluolsulfonsäure, verestert. Als Beispiele sind zu nennen: Niederalkanoyloxy, wie Acetyloxy, gegebenenfalls substituiertes Niederalkoxycarbonyloxy, wie Benzyloxycarbonyloxy, Sulfato, Halogen, wie Chlor, oder Sulfonyloxy, wie p-Toluolsulfonyloxy. Hydroxy kann nach dieser Addition an ein Oxa- oder Thiacycloalkan mit 5- bis 7 Ringatomen, wie Dihydrofuran oder Dihydropyran bzw. entsprechende Thiaanaloge, als Ester vorliegen, beispielsweise als Tetrahydrofuran- oder Tetrahydropyran-2-yloxy.

Die Reste $R_4'$ werden beispielsweise nach an sich bekannter Weise in Gruppen der Formel $-A-R_4$ überführt.

Die Ueberführung der genannten Reste $R_4'$ in solche der Formel $-A-R_4$ erfolgt in an sich bekannter Weise, beispielsweise reduktiv, wobei als Reduktionsmittel elementarer Wasserstoff in Gegenwart eines üblichen Hydrierungskatalysators, z.B. eines Palladium-, Platin-, Nickel- oder Rhodiumkatalysators, nascierender Wasserstoff, welcher z.B. durch Metalle, wie Alkalimetalle, z.B. Natrium, oder Metalllegierungen, wie Natriumamalgam, in einem protischen Lösungsmittel, wie Ammoniak oder einem Niederalkanol, z.B. Methanol, erzeugt wird, oder gegebenenfalls komplexe Hydride in Frage kommen. Solche Hydride leiten sich insbesondere von Elementen der 1. und/oder 3. Hauptgruppe ab. Die folgenden Verbindungen seien als Beispiele genannt: Lithiumaluminiumhydrid, Natriumborhydrid, Aluminiumhydrid oder Borane, welche gegebenenfalls zusammen mit einer Lewissäure, wie Aluminiumchlorid, insbesondere einem inerten Lösungsmittel, wie einem Ether, z.B. Diethylether oder Tetrahydrofruan, verwendet werden.

So lässt sich gegebenenfalls funktionell abgewandeltes Niederalkanoyloxy, Carboxy oder gegebenenfalls acetalisiertes Formyl z.B. mit Hilfe von Lithiumaluminiumhydrid, Aluminiumhydrid-Ether, oder Natriumborhydrid, funktionell abgewandeltes Carboxy und gegebenenfalls acetalisiertes Formyl, ferner mit katalytisch aktiviertem Wasserstoff und Halogen sowie Sulfonyloxy, mit Natrium in flüssigem Ammoniak, Wasserstoff oder Natriumamalgam zu Hydroxy ($-A-R_4$) reduzieren.

Funktionell abgewandeltes Hydroxy, insbesondere Niederalkanoyloxy, gegebenenfalls substituiertes Niederalkoxycarbonyloxy, Halogen, Sulfonyloxy oder 2-Tetrahydropyranyloxy lassen sich solvolytisch, z.B. hydrolytisch, gegebenenfalls in Gegenwart eines katalytischen Mittels in Hydroxy ($-A-R_4$) überführen. Niederalkanoyloxy, gegebenenfalls substituiertes Niederalkoxycarbonyloxy, Halogen bzw. Sulfonyloxy, kann beispielsweise in Gegenwart einer Base, wie eines Alkalimetallhydroxids, z.B. Natriumhydroxid, und 2-Tetrahydropyranyloxy in Gegenwart einer Säure, wie einer Mineral- oder Sulfonsäure, z.B. Schwefelsäure oder p-Toluolsulfonsäure, zu Hydroxy solvolysiert werden.

Weiterhin lässt sich funktionell abgewandeltes Hydroxy, wie verestertes Hydroxy, insbesondere Halogen oder Sulfonyloxy, durch Schwefelwasserstoff oder ein Mercaptan der Formel $H-S-R_4$ bzw. deren Alkalimetall-, wie Natriumsalze, substituieren, wobei Mercapto bzw. gegebenenfalls entsprechend substituiertes Niederalkylthio ($-S-R_4$) entsteht.

Ausgangsstoffe der Formel V, worin $R_3'$ die Gruppe der Formel $-alk-R_4'$ und $R_4'$ gegebenenfalls Carboxy oder Halogen bedeutet, werden auf üblichem Wege hergestellt. So kann man beispielsweise ein Diketon der Formel

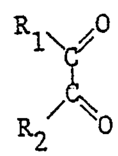

(IIIa)

mit einem Aldehyd der Formel $R_3'-C(=O)-H$ (Va) und einem Ueberschuss an Ammoniak unter Erwärmen und ohne Isolierung von Zwischenprodukten umsetzen. Dabei kann gegebenenfalls in einer vorgelagerten Reaktion ein von $R_3'$ verschiedener Rest in $R_3'$ überführt werden. Enthält beispielsweise ein von $R_3'$ verschiedener Rest $R_4'$ eine Carbamoylgruppe, so lässt sich diese beispielsweise zu Carboxy hydrolysieren. In sich gegegebenenfalls anschliessenden Reaktionen kann in Resten $R_3'$, welche gegebenenfalls verestertes Carboxy $R_4'$ aufweisen, dieses in die anderen vorstehend aufgeführten Gruppen $R_4'$ umgewandelt werden. Beispielsweise lässt sich Carboxy durch Reduktion in Formyl oder durch Halogenierung, z.B. mittels eines Phosphorhalogenids, in Halogencarbonyl überführen.

Verbindungen der Formel V, worin $R_3'$ die Gruppe der Formel $-alk-R_4'$ und $R_4'$ funktionell abgewandeltes Hydroxy bedeutet, können beispielsweise hergestellt werden, indem man an Verbindungen der Formel

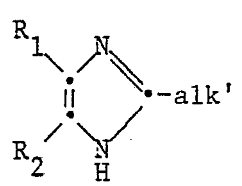

(Vb),

worin alk' Niederalkenyl darstellt, Verbindungen der Formel H-$R_4'$ (Vc) addiert.

Eine Arbeitsweise zur Ueberführung von $R_3'$ in $R_3$ besteht z.B. darin, dass man eine Verbindung der Formel

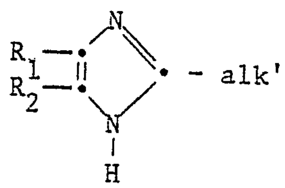

(Vd)

worin alk' Niederalkenyl bedeutet, mit einer Verbindung der Formel H-A-$R_4$ (Ve) oder ein Salz davon umsetzt.

Die Reaktion erfolgt beispielsweise in Gegenwart eines katalytischen Mittels und gegebenenfalls in einem inerten Lösungsmittel, gegebenenfalls unter Kühlen oder Erwärmen und erforderlichenfalls unter Inertgas.

Die Addition von Verbindungen der Formel Vb, worin A ein Sauerstoffatom bedeutet, wird insbesondere in Gegenwart eines sauren Mittels, wie einer Säure, beispielsweise einer Mineralsäure, z.B. Schwefelsäure, einer organischen Carbonsäure, z.B. Oxalsäure, einer Lewissäure, z.B. Bortrifluorid als Komplex, oder wie eines sauren Ionenaustauschers,

wobei beispielsweise bei einer Reaktionstemperatur von -10° bis +60°C gearbeitet wird. Die Umsetzung von Verbindungen der Formel Vb, worin A ein Schwefelatom darstellt, mit Verbindungen der Formel Va, führt zu Produkten mit Markownikoff-Oerientierung, wenn als Katalysator eine Säure, wie eine Mineralsäure, z.B. Schwefelsäure, oder eine Lewissäure, z.B. Bortrifluorid oder Aluminiumchlorid, verwendet wird. Entsprechende Verbindungen mit Anti-Markownikoff-Orientierung sind bei Einsatz radikalbildender Mittel, wie Peroxiden, z.B. Benzoylperoxid, Azoverbindungen, z.B. Azoisobuttersäuredinitril, oder von UV-Licht, erhältlich. Dabei arbeitet man beispielsweise in einem Temperaturbereich von etwa -30° bis etwa 100°C.

Die Ausgangsstoffe der Formel Va werden nach an sich bekannten Verfahren hergestellt, beispielsweise indem man eine Verbindung der Formel

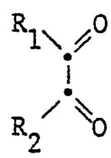

(IIIa)

mit einem Aldehyd der Formel alk'-C(=O)-H (Vf) und einem Ueberschuss an Ammoniak bei erhöhter Temperatur umsetzt.

Eine weitere, an sich bekannte Herstellungsweise für Verbindungen der Formel I oder deren Salze besteht beispielsweise darin, dass in einer Verbindung der Formel

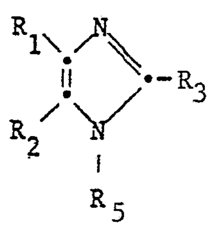

(VI),

worin $R_5$ eine in Wasserstoff überführbare Gruppe darstellt, oder einem Salz davon $R_5$ in Wasserstoff überführt und gewünschtenfalls die verfahrensgemäss erhältliche freie Verbindung in ein Salz oder ein verfahrensgemäss erhältliches Salz in die freie Verbindung oder in ein anderes Salz überführt.

Ein in Wasserstoff überführbarer Rest $R_5$ ist beispielsweise eine der üblichen geeigneten Aminoschutzgruppen. Aus der Fülle der geeigneten in Wasserstoff überführbaren Gruppen sind beispielsweise durch Sauerstoff unterbrochene, gegebenenfalls cyclische Alkyl-, gegebenenfalls substituierte Aralkyl-, Sulfenyl- oder Acylreste zu nennen. Als Alkylreste, die durch Sauerstoff unterbrochen sind, kommen z.B. Niederalkoxyniederalkyl, wie Ethoxyethyl, Phenylniederalkoxyniederalkyl, wie Benzylmethylether, Tetrahydrofuranyl oder Tetrahydropyranyl in Frage. Gegebenenfalls substituierte Aralkylreste sind z.B. solche, die im Arylteil z.B. Phenyl, Biphenyl, Anthryl und/oder Pyridyl und im Alkylteil Niederalkyl, wie Methyl oder Isopropyl, aufweisen, wie im Phenylteil durch Niederalkyl, Niederalkoxy oder Halogen substituiertes Benzyl, wie 3,5-Dimethoxy-, 2,4,6-Trimethoxy- oder 4-Brombenzyl, 2-Biphenyl-2-propyl, Di- oder Triarylmethyl, wie Diphenyl-, Triphenyl- oder $\alpha,\alpha$-Diphenyl-4-pyridylmethyl. Sulfenylreste sind beispielsweise gegebenenfalls durch Nitro substituierte Phenylsulfenylreste, wie 3-Nitrophenyl-sulfenyl. Unter Acylresten sind beispielsweise solche zu verstehen, die sich von aromatischen Carbonsäuren oder Alkancarbonsäuren sowie von Sulfonsäuren ableiten, wie gegebenenfalls substituiertes Benzoyloxycarbonyl, Niederalkanoyloxycarbonyl, z.B. tert.-Butyloxycarbonyl, Alkanoyl, wie Acetyl, oder Sulfonyl, wie p-Toluolsulfonyl.

Die Abspaltung der Amino-Schutzgruppe $R_5$ erfolgt nach an sich bekannten Methoden. Sie kann beispielsweise reduktiv, z.B. hydrogenolytisch mit gegebenenfalls nascierendem Wasserstoff, oder acidolytisch, z.B. mit Mineralsäuren, wie Halogenwasserstoffsäuren, wie Chlor- oder

Bromwasserstoffsäure, oder mit gegebenenfalls substituierten Niederalkancarbonsäuren, wie Essigsäure oder Trifluoressigsäure, erforderlichenfalls unter Kühlen oder Erwärmen, z.B. im Temperaturbereich von etwa 0° bis etwa 150°C, und in einem inerten Lösungsmittel erfolgen. Inerte Lösungsmittel sind beispielsweise Amide, wie Dimethylformamid, halogenierte Kohlenwasserstoffe, wie Chloroform oder Tetrachlorkohlenstoff, Niederalkanole, wie Methanol oder Ethanol, Ketone, wie Diniederalkylketone, z.B. Aceton, Ether, wie Tetrahydrofuran, oder Nitrile, wie Acetonitril. Insbesondere erfolgt die Ueberführung von Benzyl $R_5$ in Wasserstoff mittels Wasserstoff in Essigsäure und in Gegenwart eines Hydrierungskatalysators, wie Palladiumkohle, mit Natrium in flüssigem Ammoniak, mit Bromwasserstoffsäure in Essigsäure oder mit Fluorwasserstoff, von Triphenylmethyl $R_5$ mit Chlorwasserstoffsäure in Acetonitril, mit Trifluoressigsäure in Essigsäure oder mit Essigsäure oder von tert.-Butyl $R_5$ mit Trifluoressigsäure.

Die Ausgangsstoffe der Formel (VI) werden nach an sich bekannten Verfahren hergestellt, beispielsweise geht man von Verbindungen der Formel V oder VII aus und führt die Gruppe $R_5$ ein, beispielsweise durch übliche Aralkylierung oder Acylierung. In den entsprechend erhaltenen N-substituierten Verbindungen der Formel V wird anschliessend $R_3'$ in der vorstehend beschriebenen Weise, beispielsweise durch Oxidation oder Solvolyse, in $R_3$ übergeführt, während entsprechend N-substituierte Verbindungen der Formel VII in der nachstehend aufgeführten Weise mit Hilfe eines geeigneten Dehydrierungsmittels zu Verbindungen der Formel VI dehydrieren.

In den vorstehend beschriebenen Verfahren zur Herstellung von Verbindungen der Formeln II, IV, V, Vb und VI kann das Ammoniak, welches überwiegend im Ueberschuss zugegeben wird, auch in Form eines Ammoniak abgebenden Mittels eingesetzt werden, wobei die Freisetzung bei erhöhter Temperatur und gegebenenfalls unter Druck erfolgt. Als Ammoniak

abgebende Mittel kommen z.B. Ammoniumsalze von Niederalkancarbonsäuren, vorzugsweise Ammoniumacetat, oder einer Carbonsäure der Formel $R_3$-COOH, ferner ein geeignetes Niederalkancarbonsäureamid, insbesondere Formamid, in Frage.

Die Verbindungen der Formel I können weiterhin hergestellt werden, indem man eine Verbindung der Formel

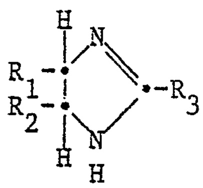

(VII)

oder ein Isomeres davon zu Verbindungen der Formel I dehydriert und gewünschtenfalls die verfahrensgemäss erhältliche freie Verbindung in ein Salz oder ein verfahrensgemäss erhältliches Salz in die freie Verbindung oder in ein anderes Salz überführt.

Verbindungen der Formel VII können beispielsweise in Form eines individuellen Stereoisomeren, optischen Isomeren wie Enantiomeren, oder als Gemische derselben, wie Racemate, sowie als geometrische (cistrans) Isomere vorliegen.

Dehydrierung von Verbindungen der Formel VII erfolgt in an sich bekannter Weise, insbesondere bei erhöhter Temperatur, beispielsweise in einem Temperaturintervall von etwa 100° bis etwa 300°C, gegebenenfalls unter Verwendung eines Dehydrierungsmittels. Als solche Mittel kommen beispielsweise Dehydrierungskatalysatoren, z.B. Nebengruppenelemente, vorzugsweise der Nebengruppe VIII, wie Palladium oder Platin, oder deren Salze, wie Ruthenium-triphenyl-phosphid-chlorid, in Betracht, wobei die Katalysatoren gegebenenfalls auf geeignetem Trägermaterial, wie Kohle, Aluminiumoxid oder Siliziumdioxid, aufgezogen sind. Weitere Dehydrierungsmittel sind beispielsweise Chinone, wie p-Benzochinone, z.B. Tetrachlor-p-benzochinon oder 2,3-Dichlor-5,6-dicyano-p-benzochinon, oder wie Anthrachinone, z.B. Phenanthren-9,10-chinon, N-Halogensuccinimide, wie N-Chlorsuccinimid, oder Manganate,

wie Bariummanganat. Die Umsetzung wird in einem inerten, gegebenenfalls hochsiedenden Lösungsmittel, wie einem Ether, z.B. Diphenylether, erforderlichenfalls unter Druck, in einem geschlossenen Gefäss und/oder unter Inertgas, z.B. Stickstoff, durchgeführt.

Die als Ausgangsstoffe zu verwendenden Verbindungen der Formel VII können beispielsweise hergestellt werden, indem man eine Verbindung der Formel

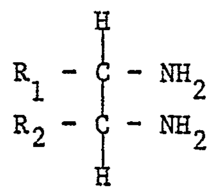

(VIIa)

oder ein Salz davon mit Verbindungen der Formel $R_3$-$Z_3$ (VIIb), worin $Z_3$ gegebenenfalls funktionell abgewandeltes Carboxy bedeutet, umsetzt. Funktionell abgewandeltes Carboxy ist unter anderem verestertes Carboxy, wie Niederalkoxycarbonyl, amidiertes Carboxy, wie gegebenenfalls substituiertes Carbamoyl, oder anhydridisiertes Carboxy, wie Halogencarbonyl.

Ein weiterer Verfahrensweg, der gegebenenfalls über Verbindungen der Formel II verläuft, besteht in der Umsetzung von Verbindungen der Formel $R_1$-$C(NH_2)$=$C(NH_2)$-$R_2$ (III l) oder deren Salzen, wie Hydrohalogeniden, mit einem gegebenenfalls funktionellen Derivat der Formel $R_3$-COOH (IIIe) oder, unter oxidierenden Bedingungen, z.B. in Gegenwart eines der vorstehend genannten Oxidationsmittel oder insbesondere Nitrobenzol, mit Verbindungen der Formel $R_3$-CHO (IIIb), gegebenenfalls unter Kühlen oder Erwärmen, und unter inerten Bedingungen. In einer bevorzugten Verfahrensweise erhitzt man Verbindungen der Formeln (III ℓ) und (IIIb) in Nitrobenzol, wobei ohne Isolierung von Zwischenprodukten direkt die entsprechende Verbindung der Formel I erhalten wird.

Der Aldehyd der Formel $R_3$-C(=O)-H (IIIb) kann in den vorstehend beschriebenen Herstellungsverfahren für Verbindungen der Formeln II, IV, und V beispielsweise auch unter den Reaktionsbedingungen aus einem Oxazinderivat der Formel

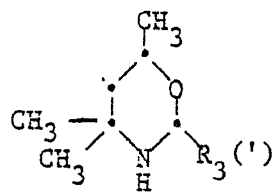

(VIII)

freigesetzt werden. Die Verbindungen der Formel VIII lassen sich beispielsweise herstellen, indem man 2-Methyl-2,4-pentandiol mit einem Nitril der Formel $R_3^{(')}$-CN in Gegenwart von Schwefelsäure umsetzt. Das dabei gebildete, entsprechend substituierte Dihydro-1,3-oxazin wird in einem Gemisch aus Tetrahydrofuran und Ethanol bei -45°C und einem pH-Wert von etwa 7 unter Einwirkung von Natriumborhydrid zu dem Tetrahydro-1,3-oxazin der Formel VIII reduziert.

Eine erfindungsgemäss erhältliche Verbindung der Formel I kann in an sich bekannter Weise in eine andere Verbindung der allgemeinen Formel I umgewandelt werden.

So kann man beispielsweise gegebenenfalls veretherte oder veresterte Hydroxygruppen ineinander überführen.

Weist beispielsweise mindestens einer der Reste $R_1$, $R_2$ und $R_3$ eine Hydroxygruppe auf, so lässt sich diese auf üblichem Wege verethern. Die Umsetzung mit einem gegebenenfalls substituierten Niederalkylierungsmittel, wie einem Niederalkanol, z.B. Methanol, in Gegenwart einer Säure, wie einer Mineralsäure, z.B. Schwefelsäure, oder eines Dehydratisierungsmittels, wie Dicyclohexylcarbodiimid, führt zu Niederalkoxy, während aromatische Alkohole beispielsweise in Gegenwart von Basen, wie Alkalimetallhydroxiden oder -carbonaten, z.B. Natriumhydroxid oder Kaliumcarbonat, mit Hilfe von Diniederalkylsulfaten oder

Diazoniederalkanen in entsprechende Niederalkylarylether überführt werden können. Umgekehrt lassen sich Ether, wie bekannt, in Alkohole spalten. So entstehen beispielsweise aus Niederalkoxyarylether durch Behandeln mit Säuren, wie mit Lewissäuren, z.B. Bortribromid, aromatische Alkohole, und Niederalkoxy kann in Gegenwart von Säuren, wie Mineralsäuren, z.B. Jodwasserstoff, in Hydroxy überführt werden.

Weiterhin lässt sich Hydroxy in Niederalkanoyloxy umwandeln, beispielsweise durch Umsetzung mit einer entsprechenden Niederalkancarbonsäure, wie Essigsäure, oder einem reaktionsfähigen Derivat davon, wie einem symmetrischen Anhydrid davon oder einem Anhydrid mit einer Halogenwasserstoffsäure, erforderlichenfalls in Gegenwart eines Kondensationsmittels, ausgehend von Anhydriden z.B. eines basischen Kondensationsmittels, wie eines Alkalimetallhydroxides oder -carbonats oder einer tertiären Stickstoffbase, z.B. eines Triamidoalkylamins oder von Pyridin, und ausgehend von einer Säure, z.B. in Gegenwart einer Säure, wie einer Protonsäure, z.B. Chlorwasserstoff-, Schwefel-, Phosphor- oder einer Sulfonsäure, oder einer Lewissäure, z.B. Bortrifluorid-Etherat. Die Hydrolyse von Niederalkanoyloxy zu Hydroxy erfolgt beispielsweise vorzugsweise durch Basenkatalyse, z.B. in Gegenwart von Natriumhydroxid.

Ferner kann man in Verbindungen der Formel I Reste $-S(O)_n-R_4$, worin n für 0 steht, zu den entsprechenden Sulfinyl- oder Sulfonylresten, worin n für 1 oder 2 steht, Reste $-S(O)_n-R_4$, worin n für 1 steht, zu den entsprechenden Sulfonylresten, worin n 2 bedeutet, und/oder Heteroarylreste $R_1$ und/oder $R_2$ mit mindestens einem freien Ringstickstoffatom $>$ N, wie Pyridylreste, N-oxidieren. Die Oxidation erfolgt vorzugsweise durch Einwirkung eines geeigneten Oxadationsmittels, vorteilhaft in einem diesem gegenüber inerten Lösungsmittel, erforderlichenfalls unter Kühlen oder Erwärmen, z.B. im Temperaturbereich von etwa -30° bis +100°C, vorzugsweise bei etwa 0° bis 60°C, in einem geschlossenen Gefäss und/oder unter Inertgas, wie Stickstoff. Geeignete Oxidationsmittel sind beispielsweise Peroxyverbindungen, wie

Wasserstoffperoxid, organische Hydroperoxide, z.B. tert.-Butylhydroperoxid, organische Persäuren, wie aromatische oder aliphatische Percarbonsäuren, z.B. m-Chlorperoxybenzoesäure, Peroxyessigsäure oder Permonophthalsäure, oxidierende Schwermetallverbindungen, wie Chrom-VI- oder Mangan-IV- bzw. Mangan-VII-verbindungen, z.B. Chromtrioxid, Chromsäure, Mangandioxid oder Kaliumpermanganat, oxidierende anorganische Sauerstoffsäuren, wie Sauerstoffsäuren des Stickstoffs, der Halogene oder Chalkogene, oder deren Anhydride oder Salze, z.B. Salpetersäure, Distickstofftetroxid, Selendioxid oder Natriummetaperjodat, ferner Ozon. Geeignete Lösungsmittel sind beispielsweise halogenierte Kohlenwasserstoffe, wie Hälogenalkane, z.B. Kohlenstofftetrachlorid, Chloroform oder Methylenchlorid, oder Carbonsäuren, wie Alkansäuren, z.B. Essigsäure, oder deren Anhydride.

In einer bevorzugten Ausführungsform dieses Oxidationsverfahrens kann man beispielsweise Thioäther der Formel I, worin n für 0 steht, und/oder einer der Reste $R_1$ und/oder $R_2$ ein unsubstituiertes Ringstickstoffatom $>$N aufweist, durch Umsetzung mit einer organischen Persäure, z.B. mit m-Chlorperbenzoesäure, in einem Halogenalkan, z.B. in Chloroform, zu den entsprechenden Sulfinyl- oder Sulfonylverbindungen, worin n für 1 oder 2 steht, und gegebenenfalls am Stickstoffatom $>$N oxidieren.

In einer anderen bevorzugten Ausführungsform kann man Thioäther der Formel I, worin n für 0 steht, durch Behandeln mit Natriummetaperjodat, vorzugsweise in einem Halogenalkan, z.B. in Kohlenstofftetrachlorid oder Chloroform, selektiv zu den entsprechenden Sulfoxiden, in denen n für 1 steht, oder diese mit Wasserstoffperoxid in Essigsäure zu Sulfonen, worin n für 2 steht, oxidieren.

Umgekehrt kann man in Verbindungen der Formel I, worin n für 1 oder 2 steht und/oder Heteroarylreste $R_1$ und/oder $R_2$ N-oxidiert sind, die Gruppe $-S(O)-R_4$ zu der entsprechenden Gruppe $S-R_4$ und/oder N-oxidierten Ringstickstoff $>$N-O zu $>$N reduzieren.

Als geeignete Reduktionsmittel eignen sich beispielsweise katalytisch aktivierter Wasserstoff, wobei Edelmetalle, wie Palladium, Platin oder Rhodium bzw. deren Oxide, als Katalysatoren verwendet werden. Weiterhin kommen reduzierende Metallkationen, wie ZinnII-, Blei II-, Kupfer I- oder Wolfram III-Verbindungen, Halogenwasserstoffe, wie Chlorwasserstoff, komplexe Metallhydride, wie Lithiumaluminium-, Tetrabutylzinn- oder Natriumborhydrid, Phosphorverbindungen, wie Phosphorhalogenide, z.B. Phosphortrichlorid, -pentachlorid oder -oxichlorid, Phosphine, z.B. Triphenylphosphin, oder Schwefelverbindungen, wie Dithionit oder Jod-Pyridin-Schwefeldioxid-Komplexe, in Frage.

Mercapto aufweisende Reste $R_3$ lassen sich, insbesondere nach Ueberführung in Alkalimetallsalzform, durch Umsetzung mit Verbindungen der Formel Hal-$R_4$, worin Hal Halogen, wie Chlor, Brom oder Jod, bedeutet und R gegebenenfalls durch Phenyl, Niederalkoxy oder Phenylniederalkoxy substituiertes Niederalkyl darstellt, alkylieren. Umgekehrt führt die Umsetzung von Thioäthern mit einem Alkalimetall, wie Natrium, in Ammoniak zu der Bildung von Mercapto.

Erhaltene freie Verbindungen der Formel I können in an sich bekannter Weise in Salze überführt werden, Saure Verbindungen, z.B. Reste $R_1$ und/oder $R_2$ gebundenes Hydroxy aufweisende Verbindungen, können z.B. durch Behandlung mit entsprechenden Basen, wie Alkalimetallhydroxiden, in Salze mit Basen überführt werden. Andererseits können basische Verbindungen der Formel I durch Behandeln mit einer der oben aufgeführten, Säureadditionssalze bildenden Säure in Säureadditionssalze umgewandelt werden.

Infolge der engen Beziehung zwischen der neuen Verbindung in freier Form und in Form ihrer Salze sind im vorausgegangenen und nachfolgend unrer der freien Verbindung oder ihren Salzen sinn- und zweckgemäss gegebenenfalls auch die entsprechenden Salze bzw. die freie Verbindung zu verstehen.

Die neue Verbindung kann, je nach der Wahl der Ausgangsstoffe und Arbeitsweisen, in Form eines der möglichen Isomeren oder als Gemische derselben vorliegen.

Die neue Verbindung einschliesslich ihrer Salze kann auch in Form ihrer Hydrate erhalten werden oder andere zur Kristallisation verwendete Lösungsmittel einschliessen.

Die neuen Ausgangsstoffe der Formeln II, IIIa, IIIb, IIIc, IIId, IIIe, IIIf, IIIi, IV, V, VI und VII sind ebenfalls Gegenstand der vorliegenden Erfindung. Bevorzugt sind diejenigen Ausgangsstoffe, die zu den bevorzugt angeführten Verbindungen der Formel I führen.

So weisen beispielsweise Verbindungen der Formel IIIi ebenfalls eine ausgeprägte antiinflammatorische Wirkung auf, insbesondere bei topischer Applikation. Diese Aktivität lässt sich beispielsweise aufgrund der Hemmwirkung auf das durch Crotonöl induzierte Ohroedem bei der Normalratte im Dosisbereich von etwa 1 bis 100 mg/ml ermitteln [Methodik: G. Tonelli, L. Thibault, Endocrinology 77, 625 (1965)]. Infolgedessen können Verbindungen der Formel IIIi als Arzneimittel, insbesondere als externe (topische) Hautphlogistika für die Behandlung entzündlicher Dermatosen, verwendet werden.

Die entsprechenden neuen Verbindungen der Formel IIIi, Verfahren zu ihrer Herstellung, pharmazeutischer Präparate, die solche Verbindungen enthalten, und ihre Verwendung, z.B. als Arzneimittel-Wirkstoffe, bilden ebenfalls einen Gegenstand der Erfindung.

Die neuen Verbindungen können, je nach der Wahl der Ausgangsstoffe und Arbeitsweisen, in Form eines der möglichen Isomeren oder als Gemische derselben, z.B. je nach der Anzahl der asymmetrischen Kohlenstoffatome, als reine optische Isomere, wie Antipoden, oder als Isomerengemische, wie Racemate, Diastereoisomerengemische oder Racematgemische, vorliegen.

Erhaltene Diastereomerengemische und Racematgemische können auf Grund der physikalisch-chemischen Unterschiede der Bestandteile in bekannter Weise in die reinen Isomeren, Diastereomeren oder Racemate aufgetrennt werden, beispielsweise durch Chromatographie und/oder fraktionierte Kristallisation. Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Ueberführung in diastereomere Salze oder Ester, z.B. durch Umsetzung eines sauren Endstoffes mit einer mit der racemischen Säure Salze bildenden optisch aktiven Base oder einer optisch aktiven Carbonsäure oder einem reaktiven Derivat davon, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Die Erfindung betrifft auch diejenigen Ausführungsformen des Verfahrens, nach denen man von einer auf irgendeiner Stufe des Verfahrens als Zwischenprodukt erhältlichen Verbindungen ausgeht und die fehlenden Schritte durchführt oder einen Ausgangsstoff in Form eines Salzes verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Verfahren zu ihrer Herstellung bilden ebenfalls einen Gegenstand der Erfindung.

Bei den erfindungsgemässen pharmazeutischen Präparaten, welche die erfindungsgemässe Verbindung oder pharmazeutisch verwendbare Salze davon enthalten, handelt es sich vorzugsweise um solche zur topischen Anwendung, ferner zur enteralen, wie oralen oder rektalen, und parenteralen Verabreichung an Warmblüter(n), wobei der pharmazeutische Wirkstoff allein oder zusammen mit einem pharmazeutisch anwendbaren Trägermaterial enthalten ist. Die tägliche Dosierung des Wirkstoffs hängt von dem Alter und dem individuellen Zustand sowie von der Applikationsweise ab. Entsprechende Mittel mit einem Konzentrationsbereich von etwa 1 bis etwa 10 % G/G, z.B. in Form von Cremen, Salben oder Lösungen, können beispielsweise 2 bis 3 x täglich appliziert werden.

Als topisch anwendbare pharmazeutische Präparate kommen in erster Linie Cremen, Salben, Pasten, Schäume, Tinkturen und Lösungen in Frage, die von etwa 0,1 bis etwa 10 % des Wirkstoffs enthalten.

Cremen sind Oel-in-Wasser Emulsionen, die mehr als 50 % Wasser aufweisen. Als ölige Grundlage verwendet man in erster Linie Fettalkohole, z.B. Lauryl-, Cetyl- oder Stearylalkohol, Fettsäuren, z.B. Palmitin- oder Stearinsäure, flüssige bis feste Wachse, z.B. Isopropylmyristat, Wollwachs oder Bienenwachs, und/oder Kohlenwasserstoffe, z.B. Vaseline (Petrolatum) oder Paraffinöl. Als Emulgatoren kommen oberflächenaktive Substanzen mit vorwiegend hydrophilen Eigenschaften in Frage, wie entsprechende nichtionische Emulgatoren, z.B. Fettsäureester von Polyalkoholen oder Ethylenoxidaddukte davon, wie Polyglycerinfessäureester

oder Polyoxyethylensorbitanfettsäureester (Tweens), ferner Polyoxyethylenfettalkoholether oder -fettsäureester, oder entsprechende ionische Emulgatoren, wie Alkalimetallsalze von Fettalkoholsulfaten, z.B. Natriumlaurylsulfat, Natriumcetylsulfat oder Natriumstearylsulfat, die man üblicherweise in Gegenwart von Fettalkoholen, z.B. Cetylalkohol oder Stearylalkohol, verwendet. Zusätze zur Wasserphase sind u.a. Mittel, welche die Austrocknung der Creme vermindern, z.B. Polyalkohole, wie Glycerin, Sorbit, Propylenglykol und/oder Polyethylenglykole, ferner Konservierungsmittel, Riechstoffe, etc.

Salben sind Wasser-in-Oel-Emulsionen, die bis zu 70 %, vorzugsweise jedoch von etwa 20 % bis etwa 50 % Wasser oder wässrige Phasen enthalten. Als Fettphase kommen in erster Linie Kohlenwasserstoffe, z.B. Vaseline, Paraffinöl und/oder Hartparaffine in Frage, die zur Verbesserung des Wasserbindungsvermögens vorzugsweise geeignete Hydroxyverbindungen, wie Fettalkohole oder Ester davon, z.B. Cetylalkohol oder Wollwachsalkohole bzw. Wollwachs, enthalten. Emulgatoren sind entsprechende lipophile Substanzen, wie Sorbitanfettsäureester (Spans), z.B. Sorbitanoleat und/oder Sorbitanisostearat. Zusätze zur Wasserphase sind u.a. Feuchthaltungsmittel, wie Polyalkohole, z.B. Glycerin, Propylenglykol, Sorbit und/oder Polyethylenglykol, sowie Konservierungsmittel, Riechstoffe, etc.

Fettsalben sind wasserfrei und enthalten als Grundlage insbesondere Kohlenwasserstoff, z.B. Paraffin, Vaseline und/oder flüssige Paraffine, ferner natürliches oder partialsynthetisches Fett, z.B. Kokosfettsäuretriglycerid, oder vorzugsweise gehärtete Oele, z.B. hydriertes Erdnuss- oder Rizinusöl, ferner Fettsäurepartialester des Glycerins, z.B. Glycerinmono- und -distearat, sowie z.B. die im Zusammenhang mit den Salben erwähnten, die Wasseraufnahmefähigkeit steigernden Fettalkohole, Emulgatoren und/oder Zusätze.

Pasten sind Cremen und Salben mit sekretabsorbierenden Puderbestandteilen, wie Metalloxiden, z.B. Titanoxid oder Zinkoxid, ferner Talk

und/oder Aluminiumsilikate, welche die Aufgabe haben, vorhandene Feuchtigkeit oder Sekrete zu binden.

Schäume werden z.B. aus Druckbehältern verabreicht und sind in Aerosolform vorliegende flüssige Oel-in-Wasser-Emulsionen, wobei halogenierte Kohlenwasserstoffe, wie Chlorfluorniederalkane, z.B. Dichlordifluormethan und Dichlortetrafluorethan, als Treibmittel verwendet werden. Als Oelphase verwendet man u.a. Kohlenwasserstoffe, z.B. Paraffinöl, Fettalkohole, z.B. Cetylalkohol, Fettsäureester, z.B. Isopropylmyristat, und/oder andere Wachse. Als Emulgatoren verwendet man u.a. Gemische von solchen mit vorwiegend hydrophilen Eigenschaften, wie Polyoxyethylen-sorbitan-fettsäureester (Tweens), und solchen mit vorwiegend lipophilen Eigenschaften, wie Sorbitanfettsäureester (Spans). Dazu kommen die üblichen Zusätze, wie Konservierungsmittel, etc.

Tinkturen und Lösungen weisen meistens eine wässerig-äthanolische Grundlage auf, der u.a. Polyalkohole, z.B. Glycerin, Glykole und/oder Polyethylenglykol, als Feuchthaltemittel zur Herabsetzung der Verdunstung, und rückfettende Substanzen, wie Fettsäureester mit niedrigen Polyethylenglykolen, d.h. im wässrigen Gemisch lösliche, lipophile Substanzen als Ersatz für die der Haut mit dem Aethanol entzogenen Fettsubstanzen, und, falls notwendig, andere Hilft- und Zusatzmittel beigegeben sind.

Die Herstellung der topisch verwendbaren pharmazeutischen Präparate erfolgt in an sich bekannter Weise, z.B. durch Lösen oder Suspendieren des Wirkstoffs in der Grundlage oder in einem Teil davon, falls notwendig. Bei Verarbeitung des Wirkstoffs als Lösung wird dieser in der Regel vor der Emulgierung in einer der beiden Phasen gelöst; bei Verarbeitung als Suspension wird er nach der Emulgierung mit einem Teil der Grundlage vermischt und dann dem Rest der Formulierung beigegeben.

Die neuen pharmazeutischen Präparate zur enteralen, wie oralen oder rektalen, und parenteralen Verabreichung enthalten z.B. von etwa 10% bis etwa 80%, vorzugsweise von etwa 20% bis etwa 60 % des Wirkstoffs. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragakanth, Methylcellulose und/oder Polyvinylpyrrolidon, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat, Hilfsmittel sind in erster Linie Fliess-, Regulier- und Schmiermittel, z.B. Kieselsäure, Talk, Stearinsäure oder Salze davon, wie Magnesium- oder Calciumsterarat, und/oder Polyethylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemische oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Oelen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositorienmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Oele, z.B. Sesamöl, oder synthetische Fettsäureester, z.B. Ethyloleat oder Triglyceride, verwendet oder wässrige Injektionssuspensionen, welche viskositätserhöhende Stoffe, z.B. Natrium-carboxymethylcellulose, Sorbit und/oder Dextran, und gegebenenfalls auch Stabilisatoren enthalten.

Die nachfolgenden Beispiele illustrieren die oben beschriebene Erfindung; sie sollen jedoch diese in ihrem Umfang in keiner Weise einschränken. Temperaturen sind in Celsiusgraden angegeben.

Beispiel 1: Zu einer Lösung von 42,2 g 1-Phenyl-2-(3-pyridyl)-glyoxal, 108 g Ammoniumacetat und 300 ml Eisessig setzt man tropfenweise während 10 Minuten bei 85-90°C unter Rühren 38,0 g 2,3,5,6-Tetrahydro-4,4,6-trimethyl-2-methylthio-methyl-1,3-oxazin zu. Man rührt weitere 20 Minuten bei 90°, kühlt ab und giesst auf 500 g Eiswasser. Die Suspension wird mit konzentriertem wässrigem Ammoniak alkalisch gestellt und mit 200 ml Methylenchlorid extrahiert. Die organische Phase wird mit Wasser gewaschen, über Magnesiumsulfat getrocknet und unter vermindertem Druck zur Trockene eingedampft. Den Rückstand chromatographiert man an 500 g Silikagel. Die Fraktionen 1-12, eluiert mit je 300 ml Chloroform—Ethylacetat (1:1), werden verworfen. Die Fraktionen 13-20, eluiert mit je 300 ml Chloroform-Ethylacetat (3:1), werden vereinigt und unter vermindertem Druck zur Trockene eingedampft. Den Rückstand kristallisiert man aus Ethylacetat-Petrolether. Das 2-Methylthiomethyl-4(5)-phenyl-5(4)-(3-pyridyl)-imidazol schmilzt bei 144-145°.

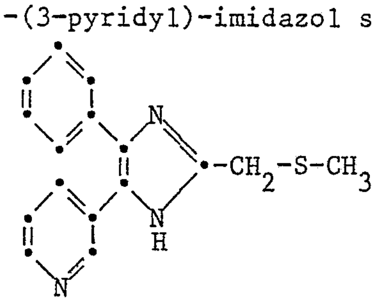

Analog wird erhalten:

2-(1-Methylthioethyl)-4(5)-phenyl-5(4)-(3-pyridyl)-imidazol, Smp. 163-165° (aus Ethylacetat-Petrolether), ausgehend von 22,8 g 1-Phenyl-2-(3-pyridyl)-glyoxal und 22,0 g 2,3,5,6-Tetrahydro-4,4,6-trimethyl-2-(1-methylthio-ethyl)-1,3-oxazin;

2-Methylthiomethyl-4,5-bis-(p-methoxyphenyl)-imidazol, Smp. 149-151° (aus Ether-Petrolether), ausgehend von 18,1 g 1,2-Bis-(p-methoxyphenyl)-glyoxal und 18,4 g 2,3,5,6-Tetrahydro-4,4,6-trimethyl-2-methylthio-methyl-1,3-oxazin.

2-(2-Methylthio-propyl)-4(5)-phenyl-5(4)-(3-pyridyl)-imidazol, Smp. 165-167° (aus Ether-Petrolether), ausgehend von 21,1 g 1-Phenyl-2-(3-pyridyl)-glyoxal und 21,7 g 2-(2,3,5,6-Tetrahydro-4,4,6-trimethyl-1,3-oxazin-2-yl)-2-methylthio-propan.

2-Ethylthiomethyl-4(5)-phenyl-5(4)-(3-pyridyl)-imidazol, Smp. 141-42°C aus THF/Hexan), ausgehend von 42,2 g 1-Phenyl-2-(3-pyridyl)-glyoxal und 40,8 g 2,3,5,6-Tetrahydro-4,4,6-trimethyl-2-ethylthiomethyl-1,3-oxazin.

2-Phenylthioethyl-4,5-(p-methoxyphenyl)-imidazol, Smp. 122-123° (aus Ethylacetat-Ether), ausgehend von 23,2 g 1,2-Bis-(p-methoxyphenyl)-glyoxal und 23,0 g 2,3,5,6-Tetrahydro-4,4,6-trimethyl-2-(2-phenylthioethyl)-1,3-oxazin.

Die Ausgangsstoffe werden wie folgt hergestellt:
338 ml konzentrierter Schwefelsäure setzt man bei 0-5° unter Rühren tropfenweise während 1 Stunde 162 g Methylthioacetonitril zu. Bei gleicher Temperatur werden noch 199,7 g 2-Methyl-2,4-pentandiol während 2 1/2 Stunden zugetropft. Man rührt 2 Stunden bei 0-5°, giesst die Lösung auf 2 l Eis/Wasser und extrahiert 4 mal mit je 250 ml Chloroform. Die wässrige Phase wird unter Eiszugabe mit konzentrierter Natronlauge bei 0-5° alkalisch gestellt. Das dabei ausgefallene Oel wird in 900 ml Ether gelöst. Die organische Phase wird 2 mal mit Sole gewaschen, dann über $MgSO_4$ getrocknet und im Vakuum eingeengt. Den Rückstand destilliert man unter vermindertem Druck. Das 5,6-Dihydro-4,4,6-trimethyl-2-methylthio-methyl-1,3-oxazin siedet bei 108-111° unter 16 Torr.

Analog wird erhalten:
5,6-Dihydro-4,4,6-trimethyl-2-(2-phenylthio-ethyl)-1,3-oxazin, Sdp. 110° (0,001 Torr), ausgehend von 59,7 g 2-Methyl-2,4-pentandiol und 88,7 g 2-Phenylthio-ethylcyanid.

Zu einer auf -75° abgekühlten Lösung von 7,48 g 5,6-Dihydro-4,4,6-trimethyl-2-methylthio-methyl-1,3-oxazin in 50 ml wasserfreiem Tetrahydrofuran tropft man unter Einleiten von Stickstoff während 1 Stunde unter Rühren eine 2-molare Lösung von Butyllithium in Hexan zu. Man rührt die Mischung eine weitere Stunde bei -75° und setzt tropfenweise eine Lösung von 2,74 ml Methyliodid in 25 ml wasserfreiem Tetrahydrofuran zu. Man entfernt das Kühlbad und lässt die Innentemperatur während 1 Stunde auf 0° ansteigen. Man giesst die Mischung auf 100 ml Eis-Wasser und stellt mit konzentrierter Salzsäure bei 0° unter Rühren auf pH 2,5. Die Suspension wird zweimal mit je 30 ml Petrolether extrahiert und anschliessend mit konzentrierter Natronlauge bei 0° auf pH 12,5 gestellt. Das ausgeschiedene Oel wird dreimal mit je 30 ml Ether extrahiert. Die vereinigten Etherphasen werden mit 20 ml konzentrierter Kochsalzlösung gewaschen, über Magnesiumsulfat getrocknet und unter vermindertem Druck zur Trockene eingedampft. Der Rückstand, ein Oel, wird mit einer Kurzwegdestillationsapparatur fraktioniert destilliert. Das 5,6-Dihydro-4,4,6-trimethyl-2-(1-methylthio-ethyl)-1,3-oxazin siedet bei 105° (16 Torr).

Analog erhält man:
2-(5,6-Dihydro-4,4,6-trimethyl-1,3-oxazin-2-yl)-2-methylthio-propan, Sdp. 34° (0,002 Torr) ausgehend von 49,8 g 5,6-Dihydro-4,4,6-trimethyl-2-(methylthio-methyl)-1,3-oxazin.

In eine Lösung aus 17,05 g 5,6-Dihydro-4,4,6-trimethyl-2-methylthio-methyl-1,3-oxazin in 91 ml Tetrahydrofuran und 95 ml 95%-igem Ethanol tropft man bei -35° gleichzeitig (aus zwei Tropftrichtern) eine Lösung von 3,45 g Natriumborhydrid in 7 ml Wasser und 1 Tropfen konzentrierter Natronlauge sowie aus dem zweiten Tropftrichter konzentrierte Salzsäure so zu, dass der pH der Reaktionsmischung 6-8 ist. Nach beendetem Zutropfen rührt man während 1 Stunde bei -35°, wobei durch Zugabe von konzentrierter Salzsäure der pH bei 6 gehalten wird.

Man setzt nun bei 0° 100 ml Eis-Wasser zu. Die ausgeschiedene Suspension wird mit konzentrierter Natronlauge auf pH 12,5 gestellt und 4 mal mit je 60 ml Ether extrahiert. Die vereinigten Etherextrakte werden mit 40 ml konzentrierter Kochsalzlösung gewaschen, über Magnesiumsulfat getrocknet und unter vermindertem Druck zur Trockene eingedampft. Das 2,3,5,6-Tetrahydro-4,4,6-trimethyl-2-methylthiomethyl-1,3-oxazin liegt als Oel vor.

Analog wird erhalten:
2,3,5,6-Tetrahydro-4,4,6-trimethyl-2-(1-methylthio-ethyl)-1,3-oxazin, Oel, ausgehend von 43,4 g 5,6-Dihydro-4,4,6-trimethyl-2-(methylthiomethyl)-1,3-oxazin, Oel, 2-Ethylthio-methyl-2,3,5,6-tetrahydro-4,4,6-trimethyl-1,3-oxazin, ausgehend von 2-Ethylthio-methyl-5,6-dihydro-4,4,6-trimethyl-1,3-oxazin, 2-(2,3,5,6-Tetrahydro-4,4,6-trimethyl-1,3-oxazin-2-yl)-2-methylthio-propan, Oel, ausgehend von 2-(5,6-Dihydro-4,4,6-trimethyl-1,3-oxazin-2-yl)-2-methylthio-propan.

2,3,5,6-Tetrahydro-4,4,6-trimethyl-2-(2-phenylthio-ethyl)-1,3-oxazin, Oel, ausgehend von 49,0 g 5,6-Dihydro-4,4,6-trimethyl-2-(2-phenylthio-ethyl)-1,3-oxazin.

Beispiel 2: Ein Gemisch aus 21,1 g 1-Phenyl-2-(3-pyridyl)-glyoxal, 11,4 g 3-Methylthio-propionaldehyd, 53,5 g Ammoniumacetat und 120 ml Eisessig wird eine Stunde unter Rückfluss gekocht und anschliessend unter Rühren in 1000 ml eines Eis-Wassergemisches gegossen. Das Gemisch wird mit konzentrierter wässriger Ammoniaklösung auf pH 8,0 gestellt. Das ausgeschiedene Oel wird zweimal mit je 500 ml Ethylacetat extrahiert. Die vereinigten organischen Phasen werden mit 200 ml Wasser und 200 ml gesättigter Kochsalzlösung gewaschen, über Magnesiumsulfat getrocknet und unter 11 Torr zur Trockene eingedampft. Der Rückstand wird an 800 g Silikagel chromatographiert. Die Fraktionen 1-11, eluiert mit je 1000 ml Methylenchlorid-Ethylacetat (25:75), werden verworfen. Die Fraktionen 12-18, eluiert mit je 1000 ml Ethylacetat-Methanol (98:2) werden vereinigt und eingedampft. Den Rückstand kristallisiert man aus Ethylacetat-Petrolether. Das 2-(2-Methylthio-ethyl)-4(5)-phenyl-5(4)-(3-pyridyl)-imidazol schmilzt bei 131-133°.

Beispiel 3: Eine Mischung aus 7,1 g α-Brombenzyl-(3-pyridyl)-keton-hydrobromid und 16,5 g Ethylthioessigsäure-Ammoniumsalz in 30 ml wasserfreiem Dimethylformamid wird unter Einleiten von Stickstoff unter Rühren während vier Stunden bei einer Innentemperatur von 100° gerührt, abgekühlt und unter 0,1 Torr bei 70° zur Trockene eingedampft. Der Rückstand wird mit 200 ml Ethylacetat und 30 ml Wasser versetzt. Diese Mischung wird mit konzentrierter wässriger Ammoniaklösung auf pH 8-9 gestellt. Die wässrige Phase wird abgetrennt und zweimal mit je 20 ml Ethylacetat extrahiert. Die Ethylacetatphasen werden vereinigt, zweimal mit je 40 ml Wasser gewaschen, über Magnesiumsulfat getrocknet und unter vermindertem Druck zur Trockene eingedampft. Den Rückstand löst man in 100 ml Chloroform-Methanol (99:1) und filtriert die Lösung durch 100 g Silikagel. Das Filtrat wird unter vermindertem Druck zur Trockene eingeengt. Den Rückstand kristallisiert man zweimal aus Ether-Petrolether. Das 2-Ethylthiomethyl-4(5)-phenyl-5(4)-(3-pyridyl)-imidazol schmilzt bei 141-142°.

Analog wird hergestellt:
2-Methylthio-methyl-4(5)-phenyl-5(4)-(3-pyridyl)-imidazol, Smp. 138-140° (aus Ether-Petrolether), ausgehend von 2,7 g α-Brom-benzyl-(3-pyridyl)-keton-hydrobromid und 5,5 g Methylthioessigsäure-Ammoniumsalz,
2-Methansulfinylmethyl-4(5)-phenyl-5(4)-(3-pyridyl)-imidazol, ausgehend von α-Brom-benzyl-(3-pyridyl)-ketonhydrobromid und Methansulfinylessigsäure-Ammoniumsalz,
2-Methoxymethyl-4(5)-phenyl-5(4)-(3-pyridyl)-imidazol, Oel, ausgehend von α-Brom-benzyl-(3-pyridyl)-keton und Methoxyessigsäure-Ammoniumsalz.

2-Methylthio-methyl-4(5)-(p-chlorphenyl)-5(4)-(3-pyridyl)-imidazol, Smp. 155-156° (aus Ethylacetat-Petrolether), ausgehend von 58,0 g α-Brom-benzyl-(3-pyridyl)-keton-hydrobromid und 58,0 g Methylthioessigsäure-Ammoniumsalz.

Der Ausgangsstoff wird wie folgt hergestellt:
Eine Lösung von 42,5 g Benzyl-(3-pyridyl)-keton in 400 ml Ethylenchlorid wird auf 50° erwärmt. Bei dieser Temperatur wird eine Lösung von 36,2 g Brom in 30 ml Ethylenchlorid zugetropft. Die Suspension wird 15 Stunden bei 50° gerührt, dann abgekühlt und filtriert. Die abfiltrierten Kristalle werden dreimal mit je 30 ml Ethylenchlorid gewaschen und unter 0,1 Torr bei 50° getrocknet. Das α-Brom-benzyl-(3-pyridyl)-keton-hydrobromid schmilzt bei 218-219,5°.

Beispiel 4: Eine Lösung von 3,5 g α-Amino-benzyl-(3-pyridyl)-keton in 50 ml wasserfreiem Tetrahydrofuran wird mit 2,0 g Methylthioessigsäurechlorid und 4 ml Diisopropylethylamin versetzt. Die Mischung wird während 20 Stunden bei 25° gerührt und anschliessend unter vermindertem Druck zur Trockene eingedampft. Den Rückstand, das N-[α-(3-Pyridylcarbonyl)-benzyl]-methylthioessigsäureamid werden mit 6,0 g Ammoniumacetat und 35 ml Eisessig drei Stunden unter Rückfluss gekocht. Dann wird die Lösung auf 70 ml konz. Ammoniak und 80 g Eis gegossen und die Suspension mit Ethylacetat extrahiert. Die organische Phase wird abgetrennt, mit gesättigter Natriumchloridlösung neutral gewaschen, über Magnesiumsulfat getrocknet und unter vermindertem Druck bei 40° eingedampft. Der Rückstand wird in 40 ml einer Ether-Ethylacetatlösung (9:1) gelöst. Die Lösung wird durch eine Schicht Kieselgel filtriert. Das Filtrat wird unter vermindertem Druck bei 40° eingeengt. Den Rückstand kristallisiert man aus Ether-Petrolether. Das 2-Methylthio-methyl-4(5)-phenyl-5(4)-(3-pyridyl)-imidazol schmilzt bei 138-140°.

Analog wird erhalten:
2-Methylthio-methyl-4(5)-phenyl-5(4)-(1-oxido-3-pyridyl)-imidazol, Oel, ausgehend von 5,6 g α-Amino-benzyl-(1-oxido-3-pyridyl)-keton und 3,9 g Methylthioessigsäurechlorid.

Die Ausgangsstoffe werden wie folgt hergestellt:
Eine Lösung von 8,5 g Benzyl-(3-pyridyl)-keton-oxim in 20 ml Pyridin wird unter Rühren auf -10° abgekühlt und tropfenweise mit einer Lösung von 7,7 g p-Toluolsulfochlorid in 15 ml Pyridin versetzt. Das Gemisch wird 24 Stunden auf 5° gekühlt und dann auf Eiswasser gegossen. Die ausgeschiedenen Kristalle werden abfiltriert, mit Wasser gewaschen und unter 0,1 Torr getrocknet. Der rohe Benzyl-(3-pyridyl)-keton-oxim-p-toluolsulfoester (Smp. 87-92°) wird in 90 ml wasserfreiem Ethanol suspendiert und bei 0° mit einer Lösung von 3,7 g Kalium-tert.-butylat in 30 ml wasserfreiem Ethanol versetzt. Das Gemisch wird 2 Stunden bei 0° gerührt. Dann wird die Suspension filtriert und das Filtrat unter vermindertem Druck bei 40° zur Trockene eingedampft. Das α-Amino-benzyl-(3-pyridyl)-keton liegt als Oel vor.

Analog wird hergestellt:
α-Amino-benzyl-(1-oxido-3-pyridyl)-keton, Oel, ausgehend von 4,2 g Benzyl-(1-oxido-3-pyridyl)-keton-oxim und 3,8 g p-Toluolsulfosäurechlorid.

Eine Lösung von 38,6 g Benzyl-(3-pyridyl)-keton in 300 ml Methylenchlorid wird auf 0° abgekühlt und mit einer Lösung von 40,0 g m-Chlorperbenzoesäure in 300 ml Methylenchlorid versetzt. Dann wird die Mischung während einer Stunde bei Raumtemperatur gerührt, nochmals mit 2,0 g m-Chlorperbenzoesäure versetzt und 10 Stunden bei Raumtemperatur gerührt. Die Mischung wird dreimal mit je 50 ml 0,5 N Natriumbikarbonatlösung und mit 50 ml Wasser extrahiert, über Magnesiumsulfat getrocknet und unter 11 Torr zur Trockene eingedampft. Der Rückstand wird an 500 g Silikagel chromatographiert. Die Fraktionen 1-5, eluiert mit je 600 ml Chloroform, werden verworfen.

Die Fraktionen 6-9, eluiert mit je 600 ml Chloroform-Methanol (19:1), werden vereinigt und unter 11 Torr zur Trockene eingedampft. Den Rückstand, das Benzyl-(1-oxido-3-pyridyl)-keton, kristallisiert man aus Eisessig. Smp. 125-126°.

Eine Suspension von 2,0 g Benzyl-(1-oxido-3-pyridyl)-keton in 8,0 ml Pyridin wird mit einer Lösung von 1,6 g Hydroxylaminhydrochlorid in 3,0 ml Pyridin versetzt. Die Mischung wird während 6 Stunden bei 100° gerührt und 15 Stunden bei Raumtemperatur stehen gelassen. Dann wird die Lösung auf 100 ml Eiswasser gegossen. Die ausgeschiedenen Kristalle werden nach 15-minütigem Stehenlassen abfiltriert, mit wenig Wasser gewaschen und unter 0,01 Torr getrocknet. Das Benzyl-(1-oxido-3-pyridyl)-keton-oxim schmilzt bei 195-197°.

Beispiel 5: Eine Lösung von 3,1 g 2-Methylthio-methyl-4(5)-phenyl-5(4)-(3-pyridyl)-imidazol in 40 ml Methylenchlorid wird auf 0° abgekühlt und mit einer Lösung von 2,11 g m-Chlorperbenzoesäure in 40 ml Methylenchlorid versetzt. Dann wird die Mischung eine Stunde bei 0° gerührt und zweimal mit je 10 ml 2 N Kaliumbikarbonatlösung und 20 ml Wasser extrahiert. Die Methylenchloridlösung wird mit Magnesiumsulfat getrocknet und unter 11 Torr zur Trockene eingedampft. Der Rückstand wird an 100 g Silikagel chromatograhpiert. Die Fraktionen 1 und 2, eluiert mit je 300 ml Chloroform, werden verworfen. Die Fraktionen 3-9, eluiert mit je 100 ml Chloroform-Methanol (99:1), werden vereinigt und unter 11 Torr zur Trockene eingedampft. Der Rückstand, das 2-Methansulfinyl-methyl-4(5)-phenyl-5(4)-(3-pyridyl)-imidazol-hemihydrat liegt als Oel (Schaum) vor.

Analog kann hergestellt werden:
2-Methansulfinyl-4(5)-(p-chlorphenyl)-5(4)-(3-pyridyl)-imidazol, gelbes Oel (Schaum), ausgehend von 3,0 g 2-Methylthio-methyl-4(5)-(p-chlorphenyl)-5(4)-(3-pyridyl)-imidazol.

2-(2-Phenylsulfinyl-ethyl)-4,5-bis-(p-methoxyphenyl)-imidazol, Oel, ausgehend von 10,0 g 2-(2-Phenylthio-ethyl)-4,5-bis-(p-methoxyphenyl)-imidazol.

Beispiel 6: Eine Lösung von 16,6 g 2-Methylthio-methyl-4(5)-phenyl-5(4)-(3-pyridyl)-imidazol in 200 ml Methylenchlorid wird auf 0° abgekühlt und unter Rühren mit einer Lösung von 11,3 g m-Chlorperbenzoesäure in 200 ml Methylenchlorid versetzt.

Die Mischung wird eine Stunde bei 0° gerührt und nochmals mit einer Lösung von 11,3 g m-Chlorperbenzoesäure in 200 ml Methylenchlorid versetzt. Die Mischung wird nochmals 1/2 Stunde bei 0° gerührt und dann zweimal mit je 50 ml 2 N Kaliumbikarbonatlösung und 50 ml Wasser extrahiert. Die Methylenchloridlösung wird abgetrennt, über Magnesiumsulfat getrocknet und unter 11 Torr zur Trockne eingedampft. Den Rückstand chromatographiert man an 500 g Silikagel. Die Fraktionen 1-4, eluiert mit je 800 ml Chloroform-Methanol (99:1), werden verworfen. Die Fraktionen 5-12, eluiert mit je 800 ml Chloroform-Methanol (99:1), werden vereinigt und unter 11 Torr zur Trockene eingedampft. Der Rückstand, das 2-Methansulfonyl-methyl-4(5)-phenyl-5(4)-(3-pyridyl)-imidazol, wird aus Methylenchlorid kristallisiert. Smp. 200-201°.

Analog wird hergestellt:
2-(2-Phenylsulfonyl-ethyl)-4,5-bis-(p-methoxyphenyl)-imidazol, Smp. 195-197° (aus Ether-Petrolether), ausgehend von 4,1 g 2-(2-Phenylthio-ethyl)-4,5-bis-(p-methoxyphenyl)-imidazol.

Beispiel 7: Eine Lösung von 27,0 g 2-Methylthio-methyl-4(5)-phenyl-5(4)-(3-pyridyl)-imidazol in 200 ml Methylenchlorid wird auf 10° abgekühlt und unter rühren mit einer Lösung von 18,4 g m-Chlorperbenzoesäure in 200 ml Methylenchlorid versetzt. Die Mischung wird eine Stunde bei 10° gerührt, nochmals mit einer Lösung von 18,4 g

m-Chlorperbenzoesäure in 200 ml Methylenchlorid versetzt und weitere zwei Stunden bei 10° gerührt. Dann wird zweimal mit je 50 ml 2 N Kaliumbikarbonatlösung und 100 ml Wasser extrahiert. Man trocknet die organische Phase mit Magnesiumsulfat und engt sie unter 11 Torr zur Trockene ein. Den Rückstand chromatographiert man an 500 g Silikagel. Die Fraktionen 1-10, eluiert mit je 1000 ml Chloroform-Methanol (19:1), werden verworfen. Die Fraktionen 11-20, eluiert mit je 1000 ml Chloroform-Methanol (9:1), werden vereinigt und unter 11 Torr zur Trockene eingedampft. Der Rückstand, das 2-Methansulfonylmethyl-4(5)-phenyl-5(4)-(1-oxido-3-pyridyl)-imidazol, wird aus Methylenchlorid-Ether kristallisiert. Smp. 207-210°.

Beispiel 8: Eine Lösung von 2,7 g 2-Methoxy-methyl-4(5)-phenyl-5(4)-(3-pyridyl)-imidazol in 30 ml Methylenchlorid wird auf 0° abgekühlt und tropfenweise unter Rühren mit einer Lösung von 2,13 g m-Chlorperbenzoesäure in 20 ml Methylenchlorid versetzt. Anschliessend wird 30 Minuten bei Raumtemperatur gerührt. Die Mischung wird nochmals mit 0,2 g m-Chlorperbenzoesäure versetzt und eine Stunde bei Raumtemperatur gerührt, zweimal mit je 30 ml 2 N Kaliumbikarbonatlösung und mit 30 ml Wasser extrahiert, über Magnesiumsulfat getrocknet und unter 11 Torr zur Trockene eingedampft. Der Rückstand chromatographiert man an 70 g Silikagel. Die Fraktionen 1-19, eluiert mit je 50 ml Chloroform, werden verworfen. Die Fraktionen 20-30, eluiert mit Chloroform-Methanol (95:5). Sie werden vereinigt und unter 11 Torr zur Trockene eingeengt. Der Rückstand, das 2-Methoxymethyl-4(5)-phenyl-5(4)-(1-oxido-3-pyridyl)-imidazol, liegt als Oel (Schaum) vor.

Beispiel 9: Eine Lösung von 2,0 g 2-[4(5)-Phenyl-5(4)-(3-pyridyl)-imidazol-2-yl]-2-methyl-propionsäureethylester in 20 ml Ethanol wird unter Rühren mit 2,0 g Natriumborhydrid versetzt. Die Mischung wird 15 Stunden bei Raumtemperatur gerührt und unter vermindertem Druck bei 50° zur Trockene eingedampft. Den Rückstand extrahiert man mit einer Mischung aus 50 ml Ethylacetat und 20 ml Wasser. Die organische

Phase wird abgetrennt, dreimal mit je 10 ml Wasser gewaschen, über Magnesiumsulfat getrocknet und unter vermindertem Druck eingedampft. Den Rückstand kristallisiert man aus Ethylacetat-Ether. Das 2-[4(5)-Phenyl-5(4)-(3-pyridyl)-imidazol-2-yl]-2-methylpropanol schmilzt bei 175-176°.

Analog wird erhalten:
2-[4(5)-Phenyl-5(4)-(1-oxido-pyridyl-3)-imidazol-2-yl]-2-methyl-propanol, Smp. 120-125°, ausgehend von 18,0 g 2-[4(5)-Phenyl-5(4)-(1-oxido-pyridyl-3)-imidazol-2-yl]-2-methyl-propionsäureethylester,
2-[4(5)-(p-Hydroxyphenyl)-5(4)-(3-pyridyl)-imidazol-2-yl]-2-methyl-propanol, Smp. 255-265°, ausgehend von 5,0 g 2-[4(5)-(p-Hydroxyphenyl)-5(4)-(3-pyridyl)-imidazol-2-yl]-2-methyl-propionsäureethyl-ester,
2-[4(5)-(p-Methoxyphenyl)-5(4)-(3-pyridyl)-imidazol-2-yl]-2-methyl-propanol, Smp. 172-178° (aus Ethylacetat-Ether), ausgehend von 2,0 g 2-[4(5)-(p-Methoxyphenyl)-5(4)-(3-pyridyl)-imidazol-2-yl]-2-methyl-propanol.

Die Ausgangsstoffe werden wie folgt hergestellt:
Eine Mischung aus 38,9 g 1-Phenyl-2-(3-pyridyl)-glyoxal, 44,6 g 2-(2,2-Dimethyl-carbethoxymethyl)-4,4,6-trimethyl-2,3,5,6-tetrahydro-1,3-oxazin, 99,1 g Ammoniumacetat und 275 ml Eisessig wird drei Stunden unter Einleiten von Stickstoff unter Rückfluss erhitzt, abgekühlt und unter Rühren in eine Mischung aus 900 g Eis und 550 ml konzentrierter wässriger Ammoniaklösung gegossen. Die Suspension wird zweimal mit je 700 ml Ethylacetat extrahiert und die organische Phase mit 500 ml Wasser gewaschen, über Magnesiumsulfat getrocknet und bei 40° unter vermindertem Druck zur Trockene eingedampft. Der Rückstand wird aus Ether kristallisiert. Der 2-[4(5)-Phenyl-5(4)-(3-pyridyl)-imidazol-2-yl]-2-methyl-propionsäureethylester schmilzt bei 134-136°.

Analog wird hergestellt:
2-[4,5-Bis-(p-methoxyphenyl)-imidazol-2-yl]-essigsäureethylester, Fp. 131-132°.

Beispiel 10: Eine Suspension von 0,58 g Lithiumaluminiumhydrid in 110 ml wasserfreiem Tetrahydrofuran wird unter Rühren und in einer Stickstoffatmosphäre bei 25° während 20 Minuten mit einer Lösung von 2,1 g 2-[4,5-Bis-(p-methoxyphenyl)-imidazol-2-yl]-essigsäureethylester in 40 ml wasserfreiem Tetrahydrofuran versetzt. Die Mischung wird 20 Stunden bei Raumtemperatur gerührt, auf 0° abgekühlt und tropfenweise mit 0,6 ml Wasser, dann mit 0,6 ml 15%iger Natronlauge und anschliessend mit 0,6 ml Wasser versetzt. Man filtriert durch Hyflo, wäscht mit 30 ml Ether nach und engt das Filtrat unter vermindertem Druck bei 40° zur Trockene ein. Den Rückstand kristallisiert man aus Ethylacetat-Petrolether. Das 2-[4,5-Bis-(p-methoxyphenyl)-imidazol-2-yl]-ethanol schmilzt bei 160-164°.

Analog wird hergestellt:
2-[4,5-Bis-(p-methoxyphenyl)-imidazol-2-yl]-propanol, ausgehend von 2,6 g 2-[4,5-Bis-(p-methoxyphenyl)-imidazol-2-yl]-propionsäureethylester.

Beispiel 11: 8,43 g 2-[4(5)-Phenyl-5(4)-(6-ethoxypyridyl-3)-imidazol-2-yl]-2-methyl-propionsäureethylester werden in 78 ml Ethanol gelöst. Anschliessend werden 3,9 g Natriumborhydrid zugegeben und die Temperatur durch Kühlen mit einem Eisbad bei 20° gehalten. Nach 5 Stunden wird das Reaktionsgemisch nochmals mit 1,2 g Natriumborhydrid versetzt und 2 Stunden bei Raumtemperatur gerührt. Anschliessend wird das Lösungsmittel unter vermindertem Druck abdestilliert. Der Rückstand wird in Wasser gelöst und mit 2-n. Salzsäure auf pH 7 eingestellt. Die so erhaltenen Kristalle werden aus Methanol-Ether umkristallisiert. Man erhält das 2-[4(5)-Phenyl-5(4)-(6-ethoxypyridyl-3)-imidazol-2-yl]-2-methyl-propanol.

Das Ausgangsmaterial wird wie folgt hergestellt.
Eine Lösung von 6,4 g Benzylcyanid in 35 ml wasserfreiem Toluol wird unter Einleiten von Stickstoff unter Rühren mit 2,4 g einer 55%-igen Natriumhydrid-Mineralöl-Dispersion versetzt. Die Suspension wird 30 Minuten bei 60° gerührt, auf 50° abgekühlt und tropfenweise mit einer Lösung von 9,75 g 2-Ethoxypyridin-5-carbonsäureethylester in 15 ml wasserfreiem Toluol versetzt. Man erhitzt die Mischung während drei Stunden auf 130°, wobei ca. 15 ml eines Toluol-Ethanol-Gemisches abdestilliert wird. Im Verlaufe der Destillation werden 15 ml wasserfreies Toluol portionenweise der Mischung zugesetzt. Anschliessend wird die Suspension abgekühlt, filtriert und die Kristalle in 100 ml Wasser gelöst. Die wässrige Lösung wird zweimal mit je 20 ml Ethylacetat gewaschen und mit 2-n Salzsäure auf pH 6-7 gestellt. Das ausgeschiedene Oel wird zweimal mit je 40 ml Ethylacetat extrahiert. Die organische Phase wird mit 20 ml Wasser gewaschen, über Magnesiumsulfat getröcknet und unter vermindertem Druck zur Trockene eingedampft. Das α-Cyanobenzyl-(6-Ethoxy-pyridyl-3)-keton liegt als Oel vor.

5,0 g α-Cyano-benzyl-(3-pyridyl)-keton werden mit 15 ml Eisessig und 15 ml konz. Schwefelsäure versetzt. Die Mischung wird unter Rühren auf 100° erwärmt, wobei Lösung eintritt. Man erhitzt die Lösung 6 Stunden bei 100°, kühlt ab und giesst auf ein Gemisch von 300 ml Eiswasser. Die ausgeschiedenen Kristalle werden abfiltriert und mit 20 ml Wasser gewaschen. Das Benzyl-(6-hydroxy-pyridyl-3)-keton schmilzt bei 172-175° (aus Methanol). Die wässrige Mutterlauge wird mit 2-n Natriumcarbonatlösung auf pH 8 gestellt. Die ausgeschiedenen Kristalle werden abfiltriert und mit wenig Wasser gewaschen. Das Benzyl-(6-ethoxy-pyridyl-3)-keton schmilzt bei 56-57°.

Eine Mischung von 5,6 g Benzyl-(6-ethoxypyridyl-3)-keton, 2,6 g sublimiertes Selendioxid und 65 ml Eisessig wird unter Rühren etwa 1 1/2 Stunden lang unter Rückfluss erhitzt. Dann kühlt man die Suspension auf 70° ab, filtriert und engt das Filtrat unter vermindertem Druck bei 50° zur Trockene ein. Den kristallinen Rückstand löst man in heissem Methanol. Die Lösung wird filtriert und unter vermindertem Druck eingeengt bis die Kristallisation einsetzt. Nach Abkühlen kann das 1-Phenyl-2-(6-ethoxypyridyl-3)-glyoxal abgenutscht werden. Dieses wird durch Filtration über Kieselgel mit Methylenchlorid als Eluiermittel gereinigt.

Analog wird hergestellt: 1-Phenyl-2-(6-hydroxypyridyl-3)-glyoxal, Smp. 224-26 °C (aus Methanol), ausgehend von 14,4 g Benzyl-(6-hydroxypyridyl-3)-keton.

Eine Mischung von 10,1 g 1-Phenyl-2-(6-ethoxypyridyl-3)-glyoxal, 10,6 g 2-[2,3,5,6-Tetrahydro-4,4,6-trimethyl-1,3-oxazinyl-2]-2-methyl-propionsäureethylester und 19,5 g Ammoniumacetat in 100 ml Eisessig werden 2 1/2 Stunden unter Rückfluss erhitzt und gerührt. Dann wird auf 70° abgekühlt und unter Rühren in eine Mischung von 450 g Eis und 225 ml konz. Ammoniak gegossen. Die dabei entstehende Suspension wird abgenutscht. Das Nutschgut wird in Essigester aufgeschlämmt und abgenutscht. Das Filtrat wird über Magnesiumsulfat getrocknet und im Vakuum eingeengt. Der Rückstand wird in Methylenchlorid gelöst und über 150 g Silikagel filtriert. Eluiermittel: Essigester. Das erhaltene Produkt wird mit dem Filterrückstand vereinigt und aus Essigester umkristallisiert. Man erhält so 2-[4(5)-Phenyl-5(4)-(6-ethoxypyridyl-3)-imidazol-2-yl]-2-methylpropionsäureethylester.

Beispiel 12: 2,6 g 2-[4(5)-Phenyl-5(4)-(6-hydroxypyridyl-3)-imidazol-2-yl]-2-methyl-propionsäureethylester werden in 26 ml Ethanol gelöst. Dann werden 1,3 g Natriumborhydrid zugegeben und die Temperatur durch Kühlen mit einem Eisbad bei 20° gehalten. Nach etwa 5 Stunden werden

nochmal 0,4 g Natriumborhydrid zugegeben und weitere 2 Stunden bei Raumtemperatur gerührt. Anschliessend destilliert man das Lösungsmittel unter vermindertem Druck ein. Der Rückstand wird in Wasser aufgenommen und mit 2-n. HCl auf pH 7 eingestellt. Die Lösung wird filtriert und der Rückstand aus Methanol-Ether umkristallisiert. Man erhält so 2-[4(5)-Phenyl-5(4)-(6-hydroxypyridyl-3)-imidazol-2-yl]-2-methyl-propanol vom Fp. 163-165°.

Das Ausgangsmaterial wird wie folgt hergestellt:
Eine Mischung von 9 g 1-Phenyl-2-(6-hydroxypyridyl-3)-glyoxal, 10,6 g 2-[2,3,5,6-Tetrahydro-4,4,6-trimethyl-1,3-oxazinyl-2]-2-methyl-propionsäureethylester und 19,5 g Ammoniumacetat in 100 ml Eisessig werden 2 1/2 Stunden lang unter Rückfluss erhitzend gerührt. Dann wird aus 70° abgekühlt und unter Rühren in eine Mischung von 400 g Eis und 200 ml konz. Ammoniak gegossen. Die dabei entstehende weisse Suspension wird abgenutscht. Das Nutschgut wird in Essigester aufgeschlämmt und abgenutscht. Das Filtrat wird über Magnesiumsulfat getrocknet und im Vakuum eingeengt. Der Rückstand wird in Methylenchlorid gelöst und über 150 g Silikagel filtriert. Eluiermittel: Essigester. Das erhaltene Produkt wird mit dem Filterrückstand vereinigt und aus Essigester umkristallisiert. Man erhält so den 2-[4(5)-Phenyl-5(4)-(6-hydroxypyridyl-3)-imidazol-2-yl]-2-methyl-propionsäureethylester, Fp. 168-172°.

Beispiel 13: Eine Mischung von 3,6 g 2-Methylthiomethyl-trans-4,5-bis-(p-methoxyphenyl)-4,5-dihydro-imidazolin und 3,7 g 2,3-Dichlor-5,6-dicyano-1,4-benzochinon in 120 ml wasserfreiem Dioxan wird während 5 Stunden unter Rückfluss erhitzt. Man kühlt ab, filtriert und engt das Filtrat unter vermindertem Druck bei 40° zur Trockene ein. Den Rückstand löst man in 100 ml Ethylacetat. Die Ethylacetatlösung wird mit 40 ml Wasser, zweimal mit je 40 ml 2 N Natriumkarbonatlösung und nochmals mit 40 ml Wasser gewaschen, über Magnesiumsulfat getrocknet, durch eine Schicht Silikagel filtriert und unter vermindertem Druck

bei 40° zur Trockene eingedampft. Das 2-Methylthiomethyl-4,5-bis-(p-methoxyphenyl)-imidazol kristallisiert aus Ether-Petrolether. Smp. 149-151°.

Das Ausgangsmaterial kann wie folgt hergestellt werden:

Eine Lösung von 5,4 g dl-1,2-Bis-(4-methoxyphenyl)-ethylendiamin und 3,0 g Methylthioessigsäureethylester in 80 ml Diphenylether wird während 3 Stunden auf 150° erhitzt. Der gebildete Ethylalkohol wird abdestilliert. Anschliessend kühlt man ab, giesst auf 200 ml Wasser und extrahiert das ausgeschiedene Oel zweimal mit je 200 ml Ether. Die vereinigten organischen Phasen werden mit 100 ml Wasser gewaschen, über Magnesiumsulfat getrocknet und unter 11 Torr bei 30° von Ether und anschliessend unter 0,1 Torr bei 60° vom restlichen Diphenylether befreit. Das 2-Methylthiomethyl-trans-4,5-bis-(p-methoxyphenyl)-4,5-dihydro-imidazolin liegt als Oel vor und wird sofort weiter umgesetzt..

<u>Beispiel 14:</u> Eine Mischung von 5,4 g 4,4'-Dimethoxystilbendiamin und 2,2 g 2-Methylthio-acetaldehyd in 100 ml Nitrobenzol wird zwei Stunden unter Rückfluss erhitzt. Die Lösung wird abgekühlt und unter 0,1 Torr bei 60° zur Trockene eingedampft. Den Rückstand chromatographiert man an 200 g Silikagel. Die Fraktionen 1-9, eluiert mit je 400 ml Methylenchlorid-Ethylacetat (25:75), werden verworgen. Die Fraktionen 10-15, eluiert mit je 400 ml Ethylacetat-Methanol (98:2), werden vereinigt und unter 11 Torr zur Trockene eingedampft. Den Rückstand kristallisiert man aus Ether-Petrolether. Das 2-Methylthio-methyl-4,5-bis-(p-methoxyphenyl)-imidazol schmilzt bei 149-151°.

<u>Beispiel 15:</u> Eine Mischung von 3,5 g α-Brom-(3-pyridyl)-benzylketon-hydrobromid und 9,68 g 3-Ethoxy-pivalinsäure-ammoniumsalz in 25 ml wasserfreiem Dimethylformamid wird unter Rühren und Einleiten von Stickstoff während 6 Stunden auf 100° erhitzt. Die Mischung wird abgekühlt und unter 11 Torr bei einer Badtemperatur von 60° zur Trockene

eingedampft. Der Rückstand wird mit 140 ml Ethylacetat und 100 ml Wasser versetzt. Der pH der Mischung wird mit konzentrierter wässriger Ammoniaklösung auf 8-9 gestellt. Die Ethylacetatlösung wird abgetrennt, zweimal mit je 30 ml Wasser gewaschen, über Magnesiumsulfat getrocknet und unter 11 Torr zur Trockene eingedampft. Der Rückstand chromatographiert man an 60 g Silikagel. Die Fraktionen 1-4, eluiert mit je 250 ml Chloroform, werden verworfen. Die Fraktionen 5-12, eluiert mit je 250 ml Chloroform-Methanol (99:2), werden vereinigt und unter 11 Torr zur Trockene eingedampft. Der Rückstand, das 2-(2-Methyl-1-ethoxy-propyl)-4(5)-phenyl-5(4)-(3-pyridyl)-imidazol, liegt als Oel vor.

Beispiel 16: 11,1 g 2-Methylthio-methyl-4,5-bis-(p-methoxyphenyl)-oxazol wird mit 97,0 g flüssigem Ammoniak und 64 g Formamid im Autoklaven während 5 Stunden auf 200° erhitzt. (Der Druck beträgt 185 atü). Man kühlt ab und giesst das Reaktionsgemisch auf 300 ml Wasser. Das ausgeschiedene Oel wird mit 150 ml Ethylacetat extrahiert. Die organische Phase wird abgetrennt, mit 30 ml gesättigter Kochsalzlösung und Wasser gewaschen, über Magnesiumsulfat getrocknet und unter vermindertem Druck zur Trockene eingedampft. Das 2-Methylthio-methyl-4,5-bis-(p-methoxyphenyl)-imidazol wird aus Ether-Petrolether kristallisiert. Smp. 149-151.

Der Ausgangsstoff kann wie folgt hergestellt werden:

Eine Lösung von 5,44 g Anisoin und 7,0 ml Triethylamin in 50 ml wasserfreiem Benzol wird bei 10° unter Rühren während einer Stunde mit einer Lösung von 2,25 g Methylthioessigsäurechlorid in 10,0 ml wasserfreiem Benzol versetzt. Die Mischung wird zwei Stunden bei 10° gerührt und anschliessend mit 60 ml Wasser versetzt. Man extrahiert zweimal mit je 60 ml Ethylacetat, trennt die organischen Phasen ab, vereinigt sie und wäscht sie mit 30 ml gesättigter wässriger Kochsalzlösung, zweimal mit je 30 ml 1 N Natriumbikarbonatlösung und nochmals mit 30 ml gesättigter Kochsalzlösung.

Die organische Phase wird dann über Magnesiumsulfat getrocknet und unter 11 Torr bei 30° zur Trockne eingeengt. Der Rückstand, der Methylthio-essigsäure-α-[(p-methoxyphenyl)-carbonyl]-(p-methoxybenzyl)-ester liegt als Oel vor.

Eine Mischung von 3,7 g Methylthio-essigsäure-α-[(p-methoxyphenyl)-carbonyl]-(p-methoxybenzyl)-ester, 1,15 g Ammoniumacetat und 100 ml Eisessig wird zwei Stunden unter Rückfluss gekocht und anschliessend unter 11 Torr bei 50° zur Trockene eingedampft. Den Rückstand chromatographiert man an 120 g Silikagel. Die Fraktionen 1-4, eluiert mit je 300 ml Benzol-Ethylacetat-Eisessig (94:5:1), werden verworfen. Die Fraktionen 5-9, eluiert mit dem gleichen Lösungsmittelgemisch, werden vereinigt und unter 11 Torr zur Trockene eingedampft. Der Rückstand enthält das 2-Methylthiomethyl-4,5-bis-(p-methoxyphenyl)-oxazol als Oel.

Beispiel 17: Eine Mischung von 4,7 g 2-Methylthiomethyl-4,5-bis-(p-methoxyphenyl)-oxazol und 20 ml Benzylamin wird im Autoklaven während 20 Stunden auf 250° erhitzt. Dann wird die Mischung abgekühlt und unter 0,1 Torr bei 50° zur Trockene eingedampft. Den Rückstand versetzt man mit Eis und extrahiert mit 100 ml Ethylacetat. Die organische Phase wird abgetrennt und zweimal mit je 20 ml 0,1-n. Salzsäure und zweimal mit je 20 ml Wasser extrahiert, über Magnesiumsulfat getrocknet und unter 11 Torr zur Trockene eingedampft. Der Rückstand, das 2-Methylthiomethyl-1-benzyl-4,5-bis-(p-methoxyphenyl)-imidazol liegt als Oel vor.

Zu einer Lösung von 2,7 g 2-Methylthiomethyl-1-benzyl-4,5-bis-(p-methoxyphenyl)-imidazol in 40 ml flüssigem Ammoniak wird solange portionenweise Natrium zugesetzt, bis die Blaufärbung der Lösung bestehen bleibt. Anschliessend wird noch 45 Minuten weitergerührt und der Natriumamid-Ueberschuss durch Zugabe von Ammoniumchlorid zersetzt.

Man entfernt das Kühlbad und lässt den Ammoniak abdampfen. Den festen Rückstand versetzt man mit 50 ml Eis-Wasser, filtriert den Rückstand ab und chromatographiert ihn an 100 g Silikagel. Die Fraktionen 1-6, eluiert mit je 300 ml Methylenchlorid-Ethylacetat (25:75), werden verworfen. Die Fraktionen 7-11, eluiert mit je 300 ml Ethylacetat-Methanol (98:2), werden vereinigt und zur Trockene eingedampft. Den Rückstand kristallisiert man aus Ether-Petrolether. Das 2-Methylthiomethyl-4,5-bis-(p-methoxyphenyl)-imidazol schmilzt bei 149-151°.

Beispiel 18: Eine Salbe, enthaltend 5% 2-Methylthio-methyl-4(5)-phenyl-5(4)-(3-pyridyl)-imidazol, kann wie folgt hergestellt werden:

Zusammensetzung:

| | |
|---|---|
| Wirkstoff | 5,0 % |
| Vaseline | 45,0 % |
| Paraffinöl | 19,6 % |
| Cetylalkohol | 5,0 % |
| Bienenwachs | 5,0 % |
| Sorbitan-sesquioleat | 5,0 % |
| p-Hydroxybenzoesäureester | 0,2 % |
| Wasser , entmineralisiert bis zu | 100,0 % |

Die Fettstoffe und Emulgatoren werden zusammengeschmolzen. Das Konservierungsmittel wird in Wasser gelöst, und die Lösung in die Fettschmelze bei erhöhter Temperatur einemulgiert. Nach dem Erkalten wird eine Suspension des Wirkstoffs in einem Teil der Fettschmelze in die Emulsion eingearbeitet.

Beispiel 19: Eine Crème, enthaltend 10% 2-Methylthio-methyl-4(5)-phenyl-5(4)-(3-pyridyl)-imidazol, kann wie folgt hergestellt werden:

Zusammensetzung:

| | |
|---|---|
| Wirkstoff | 10,0 % |
| Isopropylpalmitat | 8,0 % |
| Cetylpalmitat | 1,5 % |
| Siliconöl 100 | 0,5 % |
| Sorbitanmonostearat | 3,0 % |
| Polysorbat 60 | 3,5 % |
| 1,2-Propylenglykol PH | 20,0 % |
| Acrylsäurepolymerisat | 0,5 % |
| Triethanolamin | 0,7 % |
| Wasser, entmineralisiert bis zu | 100,0 % |

Das Acrylsäurepolymerisat wird in einem Gemisch aus entmineralisiertem Wasser und 1,2-Propylenglykol suspendiert. Unter Rühren wird hierauf Triethanolamin zugegeben. wodurch ein Schleim erhalten wird. Ein Gemisch aus Isopropylpalmitat, Cetylpalmitat, Siliconöl, Sorbitanmonostearat und Polysorbat wird auf ca. 75° erwärmt und unter Rühren in den gleichfalls auf ca. 75° erwärmten Schleim eingearbeitet. Die auf Raumtemperatur abgekühlten Crème-Grundlage wird hierauf zur Herstellung eines Konzentrates mit dem Wirkstoff verwendet. Das Konzentrat wird mittels eines Durchlaufhomogenisators homogenisiert und dann protionsweise der Grundlage hinzugefügt.

Beispiel 20: Eine Crème, enthaltend 5% 2-Methylthio-methyl-4(5)-phenyl-5(4)-(3-pyridyl)-imidazol, kann wie folgt erhalten werden:

Zusammensetzung:

| | |
|---|---|
| Wirkstoff | 5,0 % |
| Cetylpalmitat PH | 2,0 % |
| Cetylalkohol PH | 2,0 % |
| Triglyceridgemisch gesättigter mittelfettiger Fettsäuren | 5,0 % |
| Stearinsäure | 3,0 % |

| | |
|---|---|
| Glycerinstearat PH | 4,0 % |
| Cetomacrogol 1000 | 1,0 % |
| mikrokristalline Cellulose | 0,5 % |
| 1,2-Propylenglykol, dest. | 20,0 % |
| Wasser, entmineralisiert, bis zu | 100,0 % |

Cetylalkohol, Cetylpalmitat, das Triglyceridgemisch, Stearinsäure und Glycerinstearat werden zusammengeschmolzen. Die mikrokristalline Cellulose wird in einen Teil des Wassers dispergiert. Im restlichen Teil des Wassers wird Cetomacrogol gelöst und das Propylenglykol sowie der Schleim dazu gemischt. Die Fettphase wird anschliessend unter Rühren zu Wasserphase gegeben und kaltgerührt. Schliesslich wird der Wirkstoff mit einem Teil der Grundlage angerieben und hierauf die Anreibung in den Rest der Crème eingearbeitet.

Beispiel 21: Ein transparentes Hydrogel enthaltend 5% 2-Methylthiomethyl-4(5)-phenyl-5(4)-(3-pyridyl)-imidazol, kann wie folgt hergestellt werden:

Zusammensetzung:

| | |
|---|---|
| Wirkstoff | 5 % |
| Propylenglykol | 10 - 20 % |
| Isopropanol | 20 % |
| Hydroxypropyl-methylcellulose | 2 % |
| Wasser | ad 100 % |

Die Hydroxypropyl-methylcellulose wird im Wasser gequollen. Der Wirkstoff wird in einem Gemisch aus Isopropanol und Propylenglykol gelöst. Anschliessend wird die Wirkstofflösung mit einem gequollenen Cellulose-Derivat vermischt und, wenn erwünscht, mit Riechstoffen (0,1 %) versetzt.

Beispiel 22: Ein transparentes Hydrogel, enthaltend 5% 2-Methylthiomethyl-4(5)-phenyl-5(4)-(3-pyridyl)-imidazol, kann wie folgt hergestellt werden:

Zusammensetzung:

| | |
|---|---|
| Wirkstoff | 5 % |
| Propylenglykol | 20 % |
| Isopropanol | 20 % |
| Acrylsäurepolymerisat | 2 % |
| Triethanolamin | 3 % |
| Wasser | ad 100 % |

Acrylsäurepolymerisat und Wasser werden dispergiert und mit Triethanolamin neutralisiert. Der Wirkstoff wird in einem Gemisch aus Isopropanol und Propylenglykol gelöst. Anschliessend wird die Wirkstofflösung mit dem Gel vermischt, wobei, wenn erwünscht, Riechstoff (0,1 %) zugegeben werden kann.

Beispiel 23: Ein Schaumspray, enthaltend 1% 2-Methylthio-methyl-4(5)-phenyl-5(4)-(3-pyridyl)-imidazol, kann folgendermassen hergestellt werden:

Zusammensetzung:

| | |
|---|---|
| Wirkstoff | 1,00 % |
| Cetylalkohol PH | 1,70 % |
| Paraffinöl, dickflüssig | 1,00 % |
| Isopropylmyristat | 2,00 % |
| Cetomacrogol 1000 | 2,40 % |
| Sorbitanmonostearat | 1,50 % |
| 1,2-Propylenglykol PH | 5,00 % |
| Methylparaben | 0,18 % |
| Propylparaben | 0,02 % |
| Chemoderm 314 | 0,10 % |
| Wasser, entmineralisiert, bis zu | 100,00 % |

Cetylalkohol, Paraffinöl, Isopropylmyristat, Cetomacrogol und Sorbitanstearat werden zusammengeschmolzen. Methyl- und Propylparaben werden in heissem Wasser gelöst. Die Schmelze und die Lösung werden anschliessend vermischt. Der Wirkstoff, in Propylenglykol suspendiert, wird in die Grundlage eingearbeitet. Anschliessend wird Chemoderm zugeführt und mit Wasser auf das Endgewicht ergänzt.

Abfüllung:
20 ml des Gemisches wird in eine Aluminiumblockdose eingefüllt. Die Dose wird mit einem Ventil versehen, und das Treibgas wird unter Druck eingefüllt.

Beispiel 24: Tabletten, enthaltend 25 mg Wirkstoff, z.B. 2-Phenylthioethyl-4,5-bis-(p-methoxyphenyl)-imidazol oder ein Salz, z.B. das Hydrochlorid, davon können folgendermassen hergestellt werden:

Bestandteile (für 1000 Tabletten):

| | |
|---|---|
| Wirkstoff | 25,0 g |
| Lactose | 100,7 g |
| Weizstärke | 7,5 g |
| Polyethylenglykol 6000 | 5,0 g |
| Talkum | 5,0 g |
| Magnesiumstearat | 1,8 g |
| entmineralisiertes Wasser | q.s. |

Herstellung: Sämtliche feste Ingredienzien werden zunächst durch ein Sieb mit 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyethylenglykols in 100 ml Wasser hinzugegeben. Der erhaltene Stärkekleister wird zu der Hauptmenge hinzugegeben und das Gemisch, wenn

nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Beispiel 25: Kautabletten, enthaltend 30 mg Wirkstoff, z.B. Phenylsulfinylmethyl-4,5-bis-(p-methoxyphenyl)-imidazol oder ein Salz, z.B. das Hydrochlorid, davon, können z.B. folgendermassen hergestellt werden:

Zusammensetzung (für 1000 Tabletten):

| | |
|---|---|
| Wirkstoff | 30,0 g |
| Mannit | 267,0 g |
| Lactose | 179,5 g |
| Talkum | 20,0 g |
| Glycin | 12,5 g |
| Stearinsäure | 10,0 g |
| Saccharin | 1,0 g |
| 5%-ige Gleatinelösung | q.s. |

Herstellung: Alle festen Ingredienzen werden zunächst durch ein Sieb mit 0,25 mm Maschenweite getrieben. Der Mannit und die Lactose werden gemischt, unter Hinzufügen von Gelatinelösung granuliert, durch ein Sieb mit 2 mm Maschenweite getrieben, bei 50° getrocknet und nochmals durch ein Sieb mit 1,7 mm Maschenweite getrieben. Der Wirkstoff, das Glycin und das Saccharin werden sorgfältig vermischt, der Mannit, das Lactosegranulat, die Stearinsäure und das Talkum hinzugegeben, das Ganze gründlich vermischt und zu beidseitig konkaven Tabletten von etwa 100 mm Durchmesser mit Bruchrille auf der Oberseite verpresst.

Beispiel 26: Tabletten enthaltend 100 mg Wirkstoff, z.B. Phenylsulfonylethyl-4,5-bis-(p-methoxyphenyl)-imidazol oder ein Salz, z.B. das Hydrochlorid, davon, können folgendermassen hergestellt werden:

Zusammensetzung:

| | |
|---|---|
| Wirkstoff | 100,0 g |
| Lactose | 248,5 g |
| Maisstärke | 17,5 g |
| Polyethylenglykol 6000 | 5,0 g |
| Talkum | 15,0 g |
| Magnesiumstearat | 4,0 g |
| entmineralisiertes Wasser | q.s. |

Herstellung: Die festen Ingredienzen werden zunächst durch ein Sieb mit 0,6 mm Maschenweite getrieben. Dann werden Wirkstoff, Lactose, Talkum, Magnesiumstearat und die Hälfte der Stärke innig vermischt. Die andere Hälfte der Stärke wird in 65 ml Wasser suspendiert und diese Suspension zu einer siedenden Lösung des Polyethylenglykols in 260 ml Wasser hinzugegeben. Der erhaltene Kleister wird zu den pulverförmigen Substanzen hinzugefügt, das Ganze vermischt und granuliert, erforderlichenfalls unter Zugabe von Wasser. Das Granulat wird über Nacht bei 35° getrocknet, durch ein Sieb mit 1,2 mm Maschenweite getrieben und zu beidseitig konkaven Tabletten von etwa 10 mm Durchmesser mit Bruchkerbe auf der Oberseite verpresst.

Beispiel 27: Eine Lösung von 2,7 g 2-Methoxy-methyl-4(5)-phenyl-5(4)-(3-pyridyl)-oxazol in 30 ml Methylenchlorid wird auf 0° abgekühlt und tropfenweise unter Rühren mit einer Lösung von 2,1 g m-Chlorperbenzoesäure in 20 ml Methylenchlorid versetzt. Anschliessend wird 30 Minuten bei Raumtemperatur gerührt. Die Mischung wird nochmals mit 0,2 g m-Chlorperbenzoesäure versetzt und eine Stunde bei Raumtemperatur gerührt, zweimal mit je 30 ml 2 N Kaliumbikarbonatlösung und mit 30 ml Wasser extrahiert, über Magnesiumsulfat getrocknet und unter 11 Torr zur Trockene eingedampft. Der Rückstand chromatographiert man an 70 g Silikagel. Die Fraktionen 1-19, eluiert mit je 50 ml Chloroform-Methanol, werden verworfen. Die Fraktionen 20-30, eluiert mit Chloroform-Methanol (95:5). Sie werden vereinigt und unter 11 Torr zur Trockene eingeengt. Der Rückstand, das 2-Methoxy-methyl-4(5)-phenyl-5(4)-(1-oxido-3-pyridyl)-oxazol, liegt als Oel (Schaum) vor.

Das Ausgangsmaterial wird analog Beispiel 16 aus α-Hydroxybenzyl-3-pyridylketon und Methoxythioessigsäurechlorid und Ammoniak erhalten.

Beispiel 28: Analog Beispiel 16 und 27 erhält man:
2-Methansulfinyl-4(5)-(p-chlorphenyl)-5(4)-(3-pyridyl)-oxazol,
2-(2-Phenylsulfinyl-ethyl)-4,5-bis-(p-methoxyphenyl)-oxazol,
2-Methansulfonyl-methyl-4(5)-phenyl-5(4)-(3-pyridyl)-oxazol,
2-(2-Phenylsulfonyl-ethyl)- 4,5-bis-(p-methoxyphenyl)-oxazol,
2-Methansulfonyl-methyl-4(5)-phenyl-5(4)-(1-oxido-3-pyridyl)-oxazol,
2-[4(5)-Phenyl-5(4)-(3-pyridyl)-oxazol-2-yl]-2-methylpropanol,
2-[4(5)-Phenyl-5(4)-(1-oxido-pyridyl-3)-oxazol-2-yl]-2-methyl-propanol,
2-[4(5)-(p-Hydroxyphenyl)-5(4)-(3-pyridyl)-oxazol-2-yl]-2-methyl-propanol,
2-[4,5-Bis-(p-methoxyphenyl)-oxazol-2-yl]-ethanol,

2-Methylthiomethyl-4(5)-phenyl-5(4)-(3-pyridyl)-oxazol,
2-(2-Methylthio-ethyl)-4(5)-phenyl-5(4)-(3-pyridyl)-oxazol,
2-Methylthiomethyl-4,5-bis-(p-methoxyphenyl)-oxazol,
2-Ethylthiomethyl-4(5)-phenyl-5(4)-(3-pyridyl)-oxazol,
2-Methoxymethyl-4(5)-phenyl-5(4)-(3-pyridyl)-oxazol,
2-Methylthio-methyl-4(5)-(p-chlorphenyl)-5(4)-(3-pyridyl)-oxazol,
2-[4(5)-Phenyl-5(4)-(6-ethoxypyridyl-3)-oxazol-2-yl]-2-methyl-propanol,
2-[4(5)-Phenyl-5(4)-(6-hydroxypyridyl-3)-oxazol-2-yl]-methyl-propanol,
2-(2-Methyl-1-ethoxy-propyl)-4(5)-phenyl-5(4)-(3-pyridyl)-oxazol.

Patentansprüche:

1. Neue Diaryl-Imidazol-Derivate der Formel I

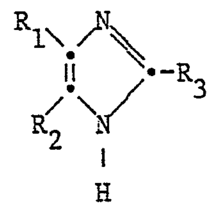

(I),

worin $R_1$ und $R_2$ unabhängig voneinander carbocyclisches Aryl und/oder Heteroaryl bedeuten, $R_3$ einen Rest der Formel $-alk-A-R_4$ darstellt, in der alk einen zweiwertigen Kohlenwasserstoffrest bedeutet, A eine Gruppe der Formel -O- oder $-S(O)_n-$ darstellt und n 0, 1 oder 2 ist, und $R_4$ einen gegebenenfalls substituierten Kohlenwasserstoffrest oder, sofern n 0 ist, Wasserstoff bedeutet, und ihre Salze.

2. Verbindungen der Formel I gemäss Anspruch 1, worin $R_1$ und $R_2$ unabhängig voneinander Phenyl-, Pyrrolyl-, Furyl-, Thienyl-, Pyridyl-, 1-Oxido-pyridyl- oder Pyrimidylreste bedeuten, die jeweils unsubstituiert oder durch Hydroxy, Halogen, Niederalkyl, Niederalkoxy und/oder Niederalkanoyloxy substituiert sind, $R_3$ eine Gruppe der Formel $-alk-A-R_4$ darstellt, in der alk Niederalkylen oder Niederalkyliden bedeutet, A eine Gruppe der Formel -O- oder $-S(O)_n-$ darstellt und n 0, 1 oder 2 ist und $R_4$ einen Phenylrest, Niederalkyl, durch einen Phenylrest, Hydroxy, Niederalkoxy, einen Phenylniederalkoxyrest und/oder Niederalkanoyloxy substituiertes Niederalkyl oder, sofern n 0 ist, Wasserstoff bedeutet, wobei ein Phenylrest jeweils unsubstituiert oder durch Hydroxy, Halogen, Niederalkyl, Niederalkoxy und/oder Niederalkanoyloxy substituiert ist, sowie ihre Salze.

3. Verbindungen der Formel I gemäss Anspruch 1, worin $R_1$ und $R_2$ unabhängig voneinander Phenyl-, Pyridyl- oder 1-Oxido-pyridylreste bedeuten, die jeweils unsubstituiert oder durch Hydroxy, Halogen

mit Atomnummer bis und mit 35, Niederalkyl mit bis und mit 4 C-Atomen, Niederalkoxy mit bis und mit 4 C-Atomen und/oder Niederalkanoyloxy mit bis und mit 5 C-Atomen substituiert sind, $R_3$ eine Gruppe der Formel $-alk-A-R_4$ darstellt, in der alk Niederalkylen mit bis und mit 4 C-Atomen oder Niederalkyliden mit bis und mit 4 C-Atomen bedeutet, A eine Gruppe der Formel -O- oder $-S(O)_n-$ darstellt und n 0, 1 oder 2 ist und $R_4$ einen Phenylrest, Niederalkyl mit bis und mit 4 C-Atomen, durch einen Phenylrest, Hydroxy, Halogen mit Atomnummer bis und mit 35, Niederalkoxy mit bis und mit 4 C-Atomen, einen Phenylniederalkoxyrest mit bis und mit 4 C-Atomen im Alkylteil und/oder Niederalkanoyloxy mit bis und mit 5 C-Atomen substituiertes Niederalkyl mit bis und mit 4 C-Atomen, wobei ein Phenylrest jeweils unsubstituiert oder durch Hydroxy, Niederalkyl mit bis und mit 4 C-Atomen, Niederalkoxy mit bis und mit 4 C-Atomen, Halogen mit Atomnummer bis und mit 35 und/oder Niederalkanoyloxy mit bis und mit 5 C-Atomen substituiert ist, oder, sofern n 0 ist, Wasserstoff darstellt, sowie ihre Salze.

4. Eine Verbindung gemäss Anspruch 1 der Formel I, worin $R_1$ und $R_2$ unabhängig voneinander gegebenenfalls durch Hydroxy, Halogen, Niederalkyl mit bis und mit 4 C-Atomen, Niederalkoxy mit bis und mit 4 C-Atomen und/oder Niederalkanoyloxy mit bis und mit 4 C-Atomen substituiertes Phenyl, Pyridyl oder 1-Oxidopyridyl bedeutet, $R_3$ einen Rest der Formel -alk-A-R darstellt, in der alk Niederalkylen mit bis und mit 4 A-Atomen, oder Niederalkyliden mit bis und mit 4 C-Atomen bedeutet, A eine Gruppe der Formel -O- oder $-S(O)_n-$ darstellt und n 0, 1 oder 2 ist und $R_4$ Phenyl, gegebenenfalls durch Phenyl, Hydroxy, Niederalkoxy mit bis und mit 4 C-Atomen und/oder Niederalkanoyloxy mit bis und mit 4 C-Atomen, substituiertes Niederalkyl mit bis und mit 4 C-Atomen darstellt und Phenyl gegebenenfalls durch Hydroxy, Halogen der Atomnummer bis und mit 35, Niederalkyl mit bis und mit 4 C-Atomen, Niederalkoxy mit bis und mit 4 C-Atomen, und/oder Niederalkanoyloxy mit bis und mit 4 C-Atomen substituiert ist, oder ein Salz davon.

5. Verbindungen der Formel I gemäss Anspruch 1, worin einerseits $R_1$ und $R_2$ durch Niederalkoxy mit bis und mit 4 C-Atomen substituiertes Phenyl bedeuten oder worin andererseits einer der Reste $R_1$ und $R_2$ Phenyl oder durch Hydroxy, Niederalkoxy mit bis und mit 4 C-Atomen oder Halogen mit Atomnummer bis und mit 35 substituiertes Phenyl und der andere Pyridyl, durch Hydroxy oder Niederalkoxy mit bis und mit 4 C-Atomen substituiertes Pyridyl oder 1-Oxido-pyridyl darstellt, $R_3$ einen Rest der Formel $-alk-A-R_4$ bedeutet, in der alk Niederalkylen mit bis und mit 4 C-Atomen oder geradkettiges bzw. verzweigtes Niederalkyliden mit bis und mit 4 C-Atomen darstellt, A eine Gruppe der Formel -O- oder $-S(O)_n-$ bedeutet und n 0, 1 oder 2 ist, und $R_4$ Phenyl, Niederalkyl mit bis und mit 4 C-Atomen oder, sofern A ein Sauerstoffatom bedeutet, Wasserstoff darstellt, sowie ihre Salze.

6. Verbindungen der Formel I gemäss Anspruch 1, worin $R_1$ und $R_2$ jeweils durch Niederalkoxy mit bis und mit 4 C-Atomen substituiertes Phenyl bedeuten, $R_3$ einen Rest der Formel $-alk-A-R_4$ darstellt, in der alk Methylen oder geradkettiges Niederalkylen mit bis und mit 4 C-Atomen bedeutet, A eine Gruppe der Formel $-S(O)_n$ darstellt, und n 0, 1 oder 2 ist, und $R_4$ Phenyl darstellt, sowie ihre Salze.

7. Verbindungen der Formel I gemäss Anspruch 1, worin einerseits $R_1$ und $R_2$ durch Niederalkoxy mit bis und mit 4 C-Atomen substituiertes Phenyl bedeuten oder worin andererseits einer der Reste $R_1$ und $R_2$ Phenyl oder durch Halogen mit Atomnummer bis und mit 35 substituiertes Phenyl und der andere Pyridyl darstellt, $R_3$ einen Rest der Formel $-alk-A-R_4$ darstellt, in der alk Methylen oder Ethylen bedeutet, A eine Gruppe der Formel -O- oder $-S(O)_n$ und n 0 oder 1 ist, und $R_4$ Phenyl oder Niederalkyl mit bis und mit 4 C-Atomen darstellt, sowie ihre Salze.

8. Eine Verbindung gemäss Anspruch 1 der Formel I, worin $R_1$ und $R_2$ durch Niederalkoxy mit bis und mit 4 C-Atomen substituiertes Phenyl oder einer der Reste $R_1$ und $R_2$ gegebenenfalls durch Hydroxy substituiertes Phenyl und der andere unsubstituiertes Pyridyl oder 1-Oxido-pyridyl bedeutet, $R_3$ einen Rest der Formel -alk-A-$R_4$ darstellt, in der alk Niederalkyliden mit bis und mit 4 C-Atomen darstellt, A ein Sauerstoff- oder Schwefelatom bedeutet, und $R_4$ Wasserstoff oder Niederalkyl mit bis und mit 2 C-Atomen darstellt, oder ein Salz davon.

9. 2-Methylthiomethyl-4(5)-phenyl-5(4)-(3-pyridyl)-imidazol oder ein Salz davon.

10. 2-(2-Methylthioethyl)-4(5)-phenyl-5(4)-(3-pyridyl)-imidazol oder ein Salz davon.

11. 2-Methylthiomethyl-4,5-bis-(p-methoxyphenyl)-imidazol oder ein Salz davon.

12. 2-Ethylthiomethyl-4(5)-phenyl-5(4)-(3-pyridyl)-imidazol oder ein Salz davon.

13. 2-Phenylthioethyl-4,5-(p-methoxyphenyl)-imidazol oder ein Salz davon.

14. 2-Methoxymethyl-4(5)-phenyl-5(4)-(3-pyridyl)-imidazol oder ein Salz davon.

15. 2-Methylthiomethyl-4(5)-(p-chlorphenyl)-5(4)-(3-pyridyl).imidazol oder ein Salz davon.

16. 2-Methylsulfinylmethyl-4(5)-(p-chlorphenyl)-5(4)-(3-pyridyl)-imidazol oder ein Salz davon.

17. 2-(2-Phenylsulfinylethyl)-4,5-bis-(p-methoxyphenyl)-imidazol oder ein Salz davon.

18. 2-Methoxymethyl-4(5)-phenyl-5(4)-(1-oxido-3-pyridyl)-imidazol oder ein Salz davon.

19. Verfahren zur Herstellung von Verbindungen der Formel I

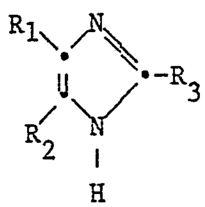

(I),

worin $R_1$ und $R_2$ unabhängig voneinander carbocyclisches Aryl und/oder Heteroaryl bedeuten, $R_3$ einen Rest der Formel -alk-A-$R_4$ darstellt, in der alk einen zweiwertigen Kohlenwasserstoffrest bedeutet, A eine Gruppe der Formel -O- oder $-S(O)_n-$ darstellt und n 0, 1 oder 2 ist, und $R_4$ einen gegebenenfalls substituierten Kohlenwasserstoffrest oder, sofern n 0 ist, Wasserstoff bedeutet, und ihrer Salze, dadurch gekennzeichnet, dass man aus einer Verbindung der Formel

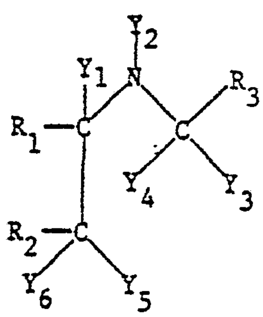

(II) ,

worin einer der Reste $Y_1$ und $Y_6$ Hydroxy oder Amino und der andere sowie $Y_2$ Wasserstoff darstellt und $Y_3$ gemeinsam mit $Y_4$ und $Y_5$ eine Gruppe der Formel =N- bedeutet oder worin $Y_1$ gemeinsam mit $Y_6$ eine Bindung darstellt, $Y_2$ Wasserstoff ist, $Y_3$ Hydroxy oder Amino bedeutet

und $Y_4$ gemeinsam mit $Y_5$ eine Gruppe der Formel -NH- darstellt oder worin $Y_1$ gemeinsam mit $Y_6$ eine Bindung darstellt, $Y_2$ gemeinsam mit $Y_3$ eine zusätzliche Bindung und einer der Reste $Y_4$ und $Y_5$ Amino und der andere Amino, Hydroxy oder reaktionsfähiges verestertes Hydroxy, insbesondere Halogen oder Sulfonyloxy, darstellt oder worin $Y_1$ Hydroxy ist, $Y_2$ sowie $Y_3$ Wasserstoff bedeutet, $Y_4$ Hydroxy oder Amino darstellt und $Y_5$ gemeinsam mit $Y_6$ eine Gruppe der Formel =NH oder, sofern $Y_4$ Amino ist, Oxo oder Imino bedeutet, oder einem Tautomeren und/oder Salz davon, unter Einführung einer gegebenenfalls zusätzlichen Bindung H-Z abspaltet oder eine Verbindung der Formel

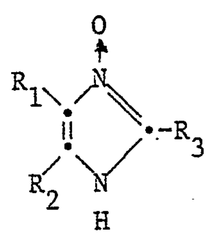 (IV)

oder ein Salz davon zu einer Verbindung der Formel I reduziert oder in einer Verbindung der Formel

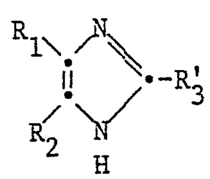 (V),

worin $R_3'$ einen in $R_3$ überführbaren Rest bedeutet, oder ein Salz davon den Rest $R_3'$ in einen Rest $R_3$ überführt oder in einer Verbindung der Formel

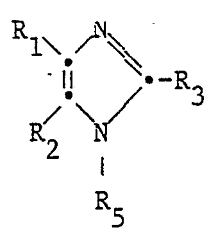 (VI),

worin $R_5$ eine in Wasserstoff überführbare Gruppe darstellt, oder einem Salz davon $R_5$ in Wasserstoff überführt oder eine Verbindung der Formel

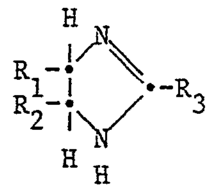

(VII)

oder ein Isomeres davon zu Verbindungen der Formel I dehydriert und gewünschtenfalls die verfahrensgemäss erhältliche freie Verbindung in ein Salz oder ein verfahrensgemäss erhältliches Salz in die freie Verbindung oder in ein anderes Salz überführt und/oder, wenn erwünscht, ein erfindungsgemäss erhältliches Gemisch von isomeren Verbindungen der Formel I in die einzelnen Isomeren auftrennt.

20. Verfahren gemäss Anspruch 19, dadurch gekennzeichnet, dass man ein Diketon der Formel

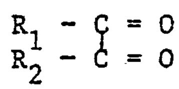

(IIIa)

mit einem Aldehyd der Formel $R_3$-C(=O)-H (IIIb) oder einem Salz davon, und einem Ueberschuss an Ammoniak unter Erwärmen umsetzt oder ein acyliertes α-Aminoketon der Formel

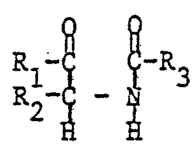

(IIIc)

oder ein Salz davon mit Ammoniak umsetzt oder eine Verbindung der Formel

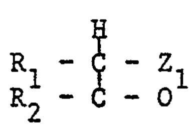

(IIIf)

worin $Z_1$ reaktionsfähiges verestertes Hydroxy bedeutet, oder ein Salz davon mit einer Verbindung der Formel

$$R_3 - Z_2 \qquad (IIIg),$$

worin $Z_2$ ein Amidinorest des Ammoniumcarboxylats bedeutet, oder ein Salz davon gegebenenfalls mit Ammoniak umsetzt oder ein Oxazol der Formel

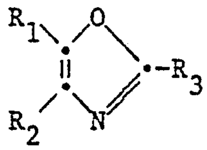

(IIIi)

mit Ammoniak über Verbindungen der Formel II zu Verbindungen der Formel I umsetzt oder eine Verbindung der Formel $R_1-C(NH_2)=C(NH_2)-R_2$ (III 1) oder ein Salz davon mit einer gegebenenfalls funktionell abgewandelten Verbindung der Formel $R_3-COOH$ (IIIe) bzw. unter oxidierenden Bedingungen, mit einer Verbindung der Formel $R_3-CHO$ (IIIb) umsetzt.

21. Die nach den Verfahren des Anspruchs 19 oder 20 erhältlichen Verbindungen.

22. Verbindungen der Formel

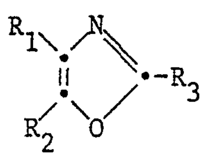

(IIIi),

worin $R_1$ und $R_2$ unabhängig voneinander carbocyclisches Aryl und/oder Heteroaryl bedeuten, $R_3$ einen Rest der Formel $-alk-A-R_4$ darstellt, in der alk einen zweiwertigen Kohlenwasserstoffrest bedeutet, A eine Gruppe der Formel -O- oder $-S(O)_n-$ darstellt und n 0, 1 oder 2 ist, und $R_4$ einen gegebenenfalls substituierten Kohlenwasserstoffrest oder, sofern n 0 ist, Wasserstoff bedeutet, und ihre Salze.

23. Verbindungen der Formel IIIi gemäss Anspruch 22, worin $R_1$ und $R_2$ unabhängig voneinander Phenyl-, Pyridyl- oder 1-Oxido-pyridylreste bedeuten, die jeweils unsubstituiert oder durch Hydroxy, Halogen mit Atomnummer bis und mit 35, Niederalkyl mit bis und mit 4 C-Atomen, Niederalkoxy mit bis und mit 4 C-Atomen und/oder Niederalkanoyloxy

mit bis und mit 5 C-Atomen substituiert sind, $R_2$ eine Gruppe der Formel -alk-A-$R_4$ darstellt, in der alk Niederalkylen mit bis und mit 4 C-Atomen oder Niederalkyliden mit bis und mit 4 C-Atomen bedeutet, A eine Gruppe der Formel -O- oder -$S(O)_n$- darstellt und n 0, 1 oder 2 ist und $R_4$ einen Phenylrest, Niederalkyl mit bis und mit 4 C-Atomen, durch einen Phenylrest, Hydroxy, Halogen mit Atomnummer bis und mit 35, Niederalkoxy mit bis und mit 4 C-Atomen, einen Phenylniederalkoxyrest mit bis und mit 4 C-Atomen im Alkylteil und/oder Niederalkanoyloxy mit bis und mit 5 C-Atomen substituiertes Niederalkyl mit bis und mit 4 C-Atomen, wobei ein Phenylrest jeweils unsubstituiert oder durch Hydroxy, Niederalkyl mit bis und mit 4 C-Atomen, Niederalkoxy mit bis und mit 4 C-Atomen, Halogen mit Atomnummer bis und mit 35 und/oder Niederalkanoyloxy mit bis und mit 5 C-Atomen substituiert ist, oder, sofern n 0 ist, Wasserstoff darstellt, sowie ihre Salze.

24. Verbindungen der Formel IIIi gemäss Anspruch 22, worin $R_1$ und $R_2$ jeweils durch Niederalkoxy mit bis und mit 4 C-Atomen substituiertes Phenyl bedeuten, $R_3$ einen Rest der Formel -alk-A-$R_4$ darstellt, in der alk Methylen oder geradkettiges Niederalkylen mit bis und mit 4 C-Atomen bedeutet, A eine Gruppe der Formel -$S(O)_n$ darstellt, und n 0, 1 oder 2 ist, und $R_4$ Phenyl darstellt, sowie ihre Salze.

25. Verbindungen der Formel IIIi gemäss Anspruch 22, worin einerseits $R_1$ und $R_2$ durch Niederalkoxy mit bis und mit 4 C-Atomen substituiertes Phenyl bedeuten oder worin andererseits einer der Reste $R_1$ und $R_2$ Phenyl oder durch Halogen mit Atomnummer bis und mit 35 substituiertes Phenyl und der andere Pyridyl darstellt, $R_3$ einen Rest der Formel -alk-A-$R_4$ darstellt, in der alk Methylen oder Ethylen bedeutet, A eine Gruppe der Formel -O- oder -$S(O)_n$ und n 0 oder 1 ist, und $R_4$ Phenyl oder Niederalkyl mit bis und mit 4 C-Atomen darstellt, sowie ihre Salze.

26. 2-Methylthioethyl-4(5)-phenyl-5(4)-(3-pyridyl)-oxazol oder ein Salz davon.

27. 2-Methoxymethyl-4(5)-phenyl-5(4)-(3-pyridyl)-oxazol oder ein Salz davon.

28. 2-(2-Phenylsulfonylethyl)-4(5)-phenyl-5(4)-(3-pyridyl)-oxazol oder ein Salz davon.

29. Verbindungen gemäss einem der Ansprüche 1-18 mit externer Haut-phlogostatischer Wirkung, mit Anti-Herpes-Wirkung und/oder mit Wirkung als Sonnenschutzmittel.

30. Verbindungen der Formel IIIi gemäss einem der Ansprüche 22-28 mit Haut-phlogostatischer und/oder antithrombotischer Wirkung.

31. Verwendung von Verbindungen gemäss einem der Ansprüche 1-18 und 22-28 zur Herstellung von Arzneimitteln.

32. Verbindungen gemäss einem der Ansprüche 1,2,4,8, 9-12 und 14 zur Anwendung in einem Verfahren zur therapeutischen Behandlung des tierischen oder menschlichen Körpers.

33. Verbindungen gemäss einem der Ansprüche 3, 5-7, 13, 15-18 und 22-28 zur Anwendung in einem Verfahren zur therapeutischen Behandlung des tierischen oder menschlichen Körpers.

34. Pharmazeutische Präparate, enthaltend eine Verbindung gemäss einem der Ansprüche 1,2,4,8-12 und 14 in freier Form eines pharmazeutisch verwendbaren Salzes neben üblichen Träger- und Hilfsstoffen.

35. Pharmazeutische Präparate, enthaltend eine Verbindung gemäss einem der Ansprüche 3, 5-7, 13, 15-18 und 22-28 in freier Form eines pharamzeutisch verwendbaren Salzes neben üblichen Träger- und Hilfsstoffen.

36. Verfahren zur Herstellung von Verbindung der Formel

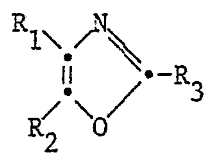

(IIIi),

worin $R_1$ und $R_2$ unabhängig voneinander carbocyclisches Aryl und/oder Heteroaryl bedeuten, $R_3$ einen Rest der Formel $-alk-A-R_4$ darstellt, in der alk einen zweiwertigen Kohlenwasserstoffrest bedeutet, A eine Gruppe der Formel -O- oder $-S(O)_n-$ darstellt und n 0, 1 oder 2 ist, und $R_4$ einen gegebenenfalls substituierten Kohlenwasserstoffrest oder, sofern n 0 ist, Wasserstoff bedeutet, und ihrer Salze, dadurch gekennzeichnet, dass man Verbindungen der Formel

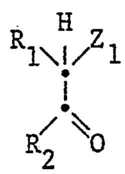

(IIIf),

worin $Z_1$ gegebenenfalls reaktionsfähiges verestertes Hydroxy bedeutet, mit Carbonsäuren der Formel $R_3$-COOH (IIIe),deren funktionelle Derivate oder deren Salze und mit Ammoniak umsetzt oder Verbindungen der Formel

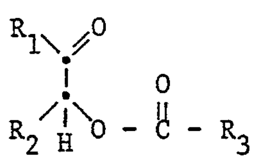

(IIIk),

welche durch Umsetzung von Verbindungen der Formel (IIIf) mit gegebenenfalls funktionell abgewandelten Derivaten von Verbindungen der Formel (IIIe) erhältlich sind, mit Ammoniak umsetzt, gewünschtenfalls in einer so erhaltenen Verbindung der Formel (IIIi) nicht N-oxidierte Heteroaryl-Reste $R_1$ bzw. $R_2$ mit Hilfe eines Oxidationsmittels N-oxidiert und gewünschtenfalls die verfahrensgemäss erhältliche freie

Verbindung in ein Salz oder ein verfahrensgemäss erhältliche freie Verbindung in ein Salz oder ein verfahrensgemäss erhältliches Salz in die freie Verbindung oder in ein anderes Salz überführt und/oder, wenn erwünscht, ein erfindungsgemäss erhältliches Gemisch von isomeren Verbindungen der Formel I in die einzelnen Isomeren auftrennt.